# EUROPEAN PATENT SPECIFICATION

(11) **EP 1 928 504 B1**
(45) Date of publication and mention of the grant of the patent: **24.04.2013**
(21) Application number: 06774831.9
(22) Date of filing: 18.08.2006
(51) Int. Cl.: A61K 48/00, A61P 3/10

(54) **LIVER-DIRECTED GENE THERAPY**
GEGEN DIE LEBER GERICHTETE GENTHERAPIE
THERAPIE DE GENE DANS LE FOIE

(30) Priority: 19.08.2005 AU 2005904525; 14.03.2006 AU 2006901311
(43) Date of publication of application: 11.06.2008
(73) Proprietor: University of Technology, Sydney, Broadway NSW 2007 (AU)
(72) Inventor: SIMPSON, Ann, Margaret, Killarney Heights, NSW 2087 (AU); REN, Binhai, St Leonards, NSW 2065 (AU)
(74) Representative: Elsy, David
(86) International application number: PCT/AU2006/001195
(87) International publication number: WO 2007/019646

(56) References cited:
- WO-A-02/092134
- US-B1- 6 218 186
- COSSET F-L ET AL: "HIGH-TITER PACKAGING CELLS PRODUCING RECOMBINANT RETROVIRUSES RESISTANT TO HUMAN SERUM" JOURNAL OF VIROLOGY, THE AMERICAN SOCIETY FOR MICROBIOLOGY, US, vol. 69, no. 12, 1 December 1995 (1995-12-01), pages 7430-7436, XP000569527 ISSN: 0022-538X
- PODEVIN G. ET AL.: 'Factors influencing immune response after in vivo retrovirus-mediated gene transfer to the liver' J. GENE MED. vol. 6, 2004, pages 16 - 21, XP003008953
- ALVES A. ET AL.: 'Total vascular exclusion of the liver enhances the efficacy of retroviral-mediated associated thymidine kinase and interleukin-2 genes transfer against multiple hepatic tumors in rats' SURGERY vol. 133, no. 6, June 2003, pages 669 - 677, XP008124867

### SEQUENCE LISTING

<110> University of Technology, Sydney
<120> Liver-directed gene therapy
<130> 684600C
<150> AU 2005904525
   <151> 2005-08-19
<150> AU 2006901311
   <151> 2006-03-14
<160> 39
<170> PatentIn version 3.2
<210> 1
   <211> 25
   <212> DNA
   <213> recombinant
<400> 1
   caacagccac aacgtctata tcatg 25
<210> 2
   <211> 21
   <212> DNA
   <213> recombinant
<400> 2
   atgttgtggc ggatcttgaa g 21
<210> 3
   <211> 20
   <212> DNA
   <213> recombinant
<400> 3
   agcctttgtg aaccaacacc 20
<210> 4
   <211> 20
   <212> DNA
   <213> recombinant
<400> 4
   ccagttggta gagggagcag 20
<210> 5
   <211> 20
   <212> DNA
   <213> Rattus sp.
<400> 5
   agaaagggag ggagtcagag 20
<210> 6
   <211> 20
   <212> DNA
   <213> Rattus sp.
<400> 6
   caccaggaag tatgggagtc 20
<210> 7
   <211> 20
   <212> DNA
   <213> Rattus sp.
<400> 7
   aaacgccaca caaggagaac 20
<210> 8
   <211> 20
   <212> DNA
   <213> Rattus sp.
<400> 8
   ctgttatggg accgctcaag 20
<210> 9
   <211> 21
   <212> DNA
   <213> Rattus sp.
<400> 9
   ggactttctt gcctgagcag a 21
<210> 10
   <211> 21
   <212> DNA
   <213> Rattus sp.
<400> 10
   aactcggtgg atggttcgtg t 21
<210> 11
   <211> 20
   <212> DNA
   <213> Rattus sp.
<400> 11
   atggccctgt ggatgcgctt 20
<210> 12
   <211> 22
   <212> DNA
   <213> Rattus sp.
<400> 12
   tagttgcagt agttctccag ct 22
<210> 13
   <211> 20
   <212> DNA
   <213> Rattus sp.
<400> 13
   atggcccgtg ggatccgctt 20
<210> 14
   <211> 20
   <212> DNA
   <213> Rattus sp.
<400> 14
   tgccaaggtc tgaaggtcac 20
<210> 15
   <211> 20
   <212> DNA
   <213> Rattus sp.
<400> 15
   gtttctgcag aagtctctgg 20
<210> 16
   <211> 20
   <212> DNA
   <213> Rattus sp.
<400> 16
   agttcttgca gccagctttg 20
<210> 17
   <211> 18
   <212> DNA
   <213> Rattus sp.
<400> 17
   gaccgtttac gtggctgg 18
<210> 18
   <211> 24
   <212> DNA
   <213> Rattus sp.
<400> 18
   cggttcctct tggtgttcat caac 24
<210> 19
   <211> 24
   <212> DNA
   <213> Rattus sp.
<400> 19
   tgaacagagg gctcaatacg aaac 24
<210> 20
   <211> 24
   <212> DNA
   <213> Rattus sp.
<400> 20
   agacagaagg gaggctacaa atcc 24
<210> 21
   <211> 18
   <212> DNA
   <213> Rattus sp.
<400> 21
   cacgcaggtc aagatctg 18
<210> 22
   <211> 20
   <212> DNA
   <213> Rattus sp.
<400> 22
   tgcccgcctg gaaggtggcg 20
<210> 23
   <211> 24
   <212> DNA
   <213> Rattus sp.
<400> 23
   atgggaagag aaaacacacc agac 24
<210> 24
   <211> 24
   <212> DNA
   <213> Rattus sp.
<400> 24
   taatcgtcgt cgtcctcctc gttc 24
<210> 25
   <211> 24
   <212> DNA
   <213> Rattus sp.
<400> 25
   tggctttctg tcccttctgt gagg 24
<210> 26
   <211> 24
   <212> DNA
   <213> Rattus sp.
<400> 26
   tccaagactc ctgtgcggta gtag 24
<210> 27
   <211> 23
   <212> DNA
   <213> Rattus sp.
<400> 27
   aagagtggcg actccagaag ttg 23
<210> 28
   <211> 25
   <212> DNA
   <213> Rattus sp.
<400> 28
   accacacctg tatccttgct tcagg 25
<210> 29
   <211> 16
   <212> DNA
   <213> Rattus sp.
<400> 29
   ttagcaactg ggtctg 16
<210> 30
   <211> 20
   <212> DNA
   <213> Rattus sp.
<400> 30
   ggtgtagtcc tacactcatg 20
<210> 31
   <211> 20
   <212> DNA
   <213> Rattus sp.
<400> 31
   aagggaacaa catcgtagga 20
<210> 32
   <211> 20
   <212> DNA
   <213> Rattus sp.
<400> 32
   cattggcggt cttcatagta 20
<210> 33
   <211> 20
   <212> DNA
   <213> Rattus sp.
<400> 33
   cgtaaagacc tctatgccaa 20
<210> 34
   <211> 20
   <212> DNA
   <213> Rattus sp.
<400> 34
   agccatgcca aatgtctcat 20
<210> 35
   <211> 20
   <212> DNA
   <213> Rattus sp.
<400> 35
   gtccctggag cattttcccg 20
<210> 36
   <211> 21
   <212> DNA
   <213> Rattus sp.
<400> 36
   ctgaggaact cttacctccg c 21
<210> 37
   <211> 10380
   <212> DNA
   <213> recombinant
<220>
   <221> misc_feature
   <222> (9967)..(9967)
   <223> n is a, c, g, or t
<400> 37
   attaattccg tgtattctat agtgtcacct aaatcgtatg tgtatgatac ataaggttat     60
   gtattaattg tagccgcgtt ctaacgacaa tatgtacaag cctaattgtg tagcatctgg    120
   cttactgaag cagaccctat catctctctc gtaaactgcc gtcagagtcg gtttggttgg    180
   acgaaccttc tgagtttctg gtaacgccgt cccgcacccg gaaatggtca gcgaaccaat    240
   cagcagggtc atcgctagcc agatcctcta cgccggacgc atcgtggccg gcatcaccgg    300
   cgccacaggt gcggttgctg gcgcctatat cgccgacatc accgatgggg aagatcgggc 360
   tcgccacttc gggctcatga gcgcttgttt cggcgtgggt atggtggcag gccccgtggc 420
   cgggggactg ttgggcgcca tctccttgca tgcaccattc cttgcggcgg cggtgctcaa 480
   cggcctcaac ctactactgg gctgcttcct aatgcaggag tcgcataagg gagagcgtcg 540
   aatggtgcac tctcagtaca atctgctctg atgccgcata gttaagccag ccccgacacc 600
   cgccaacacc cgctgacgcg ccctgacggg cttgtctgct cccggcatcc gcttacagac 660
   aagctgtgac cgtctccggg agctgcatgt gtcagaggtt ttcaccgtca tcaccgaaac 720
   gcgcgagacg aaagggcctc gtgatacgcc tatttttata ggttaatgtc atgataataa 780
   tggtttctta gacgtcaggt ggcacttttc ggggaaatgt gcgcggaacc cctatttgtt 840
   tatttttcta aatacattca aatatgtatc cgctcatgag acaataaccc tgataaatgc 900
   ttcaataata ttgaaaaagg aagagtatga gtattcaaca tttccgtgtc gcccttattc 960
   ccttttttgc ggcattttgc cttcctgttt ttgctcaccc agaaacgctg gtgaaagtaa 1020
   aagatgctga agatcagttg ggtgcacgag tgggttacat cgaactggat ctcaacagcg 1080
   gtaagatcct tgagagtttt cgccccgaag aacgttttcc aatgatgagc acttttaaag 1140
   ttctgctatg tggcgcggta ttatcccgta ttgacgccgg gcaagagcaa ctcggtcgcc 1200
   gcatacacta ttctcagaat gacttggttg agtactcacc agtcacagaa aagcatctta 1260
   cggatggcat gacagtaaga gaattatgca gtgctgccat aaccatgagt gataacactg 1320
   cggccaactt acttctgaca acgatcggag gaccgaagga gctaaccgct tttttgcaca 1380
   acatggggga tcatgtaact cgccttgatc gttgggaacc ggagctgaat gaagccatac 1440
   caaacgacga gcgtgacacc acgatgcctg tagcaatggc aacaacgttg cgcaaactat 1500
   taactggcga actacttact ctagcttccc ggcaacaatt aatagactgg atggaggcgg 1560
   ataaagttgc aggaccactt ctgcgctcgg cccttccggc tggctggttt attgctgata 1620
   aatctggagc cggtgagcgt gggtctcgcg gtatcattgc agcactgggg ccagatggta 1680
   agccctcccg tatcgtagtt atctacacga cggggagtca ggcaactatg gatgaacgaa 1740
   atagacagat cgctgagata ggtgcctcac tgattaagca ttggtaactg tcagaccaag 1800
   tttactcata tatactttag attgatttaa aacttcattt ttaatttaaa aggatctagg 1860
   tgaagatcct ttttgataat ctcatgacca aaatccctta acgtgagttt tcgttccact 1920
   gagcgtcaga ccccgtagaa aagatcaaag gatcttcttg agatcctttt tttctgcgcg 1980
   taatctgctg cttgcaaaca aaaaaaccac cgctaccagc ggtggtttgt ttgccggatc 2040
   aagagctacc aactcttttt ccgaaggtaa ctggcttcag cagagcgcag ataccaaata 2100
   ctgtccttct agtgtagccg tagttaggcc accacttcaa gaactctgta gcaccgccta 2160
   catacctcgc tctgctaatc ctgttaccag tggctgctgc cagtggcgat aagtcgtgtc 2220
   ttaccgggtt ggactcaaga cgatagttac cggataaggc gcagcggtcg ggctgaacgg 2280
   ggggttcgtg cacacagccc agcttggagc gaacgaccta caccgaactg agatacctac 2340
   agcgtgagca ttgagaaagc gccacgcttc ccgaagggag aaaggcggac aggtatccgg 2400
   taagcggcag ggtcggaaca ggagagcgca cgagggagct tccaggggga aacgcctggt 2460
   atctttatag tcctgtcggg tttcgccacc tctgacttga gcgtcgattt ttgtgatgct 2520
   cgtcaggggg gcggagccta tggaaaaacg ccagcaacgc ggccttttta cggttcctgg 2580
   ccttttgctg gccttttgct cacatgttct ttcctgcgtt atcccctgat tctgtggata 2640
   accgtattac cgcctttgag tgagctgata ccgctcgccg cagccgaacg accgagcgca 2700
   gcgagtcagt gagcgaggaa gcggaagagc gcccaatacg caaaccgcct ctccccgcgc 2760
   gttggccgat tcattaatgc agctgtggaa tgtgtgtcag ttagggtgtg gaaagtcccc 2820
   aggctcccca gcaggcagaa gtatgcaaag catgcatctc aattagtcag caaccaggtg 2880
   tggaaagtcc ccaggctccc cagcaggcag aagtatgcaa agcatgcatc tcaattagtc 2940
   agcaaccata gtcccgcccc taactccgcc catcccgccc ctaactccgc ccagttccgc 3000
   ccattctccg ccccatggct gactaatttt ttttatttat gcagaggccg aggccgcctc 3060
   ggcctctgag ctattccaga agtagtgagg aggctttttt ggaggcctag gcttttgcaa 3120
   aaagcttgga cacaagacag gcttgcgaga tatgtttgag aataccactt tatcccgcgt 3180
   cagggagagg cagtgcgtaa aaagacgcgg actcatgtga aatactggtt tttagtgcgc 3240
   cagatctcta taatctcgcg caacctattt tcccctcgaa cactttttaa gccgtagata 3300
   aacaggctgg gacacttcac atgagcgaaa aatacatcgt cacctgggac atgttgcaga 3360
   tccatgcacg taaactcgca agccgactga tgccttctga acaatggaaa ggcattattg 3420
   ccgtaagccg tggcggtctg taccgggtgc gttactggcg cgtgaactgg gtattcgtca 3480
   tgtcgatacc gtttgtattt ccagctacga tcacgacaac cagcgcgagc ttaaagtgct 3540
   gaaacgcgca gaaggcgatg gcgaaggctt catcgttatt gatgacctgg tggataccgg 3600
   tggtactgcg gttgcgattc gtgaaatgta tccaaaagcg cactttgtca ccatcttcgc 3660
   aaaaccggct ggtcgtccgc tggttgatga ctatgttgtt gatatcccgc aagatacctg 3720
   gattgaacag ccgtgggata tgggcgtcgt attcgtcccg ccaatctccg gtcgctaatc 3780
   ttttcaacgc ctggcactgc cgggcgttgt tctttttaac ttcaggcggg ttacaatagt 3840
   ttccagtaag tattctggag gctgcatcca tgacacaggc aaacctgagc gaaaccctgt 3900
   tcaaaccccg ctttaaacat cctgaaacct cgacgctagt ccgccgcttt aatcacggcg 3960
   cacaaccgcc tgtgcagtcg gcccttgatg gtaaaaccat ccctcactgg tatcgcatga 4020
   ttaaccgtct gatgtggatc tggcgcggca ttgacccacg cgaaatcctc gacgtccagg 4080
   cacgtattgt gatgagcgat gccgaacgta ccgacgatga tttatacgat acggtgattg 4140
   gctaccgtgg cggcaactgg atttatgagt gggccccgga tctttgtgaa ggaaccttac 4200
   ttctgtggtg tgacataatt ggacaaacta cctacagaga tttaaagctc taaggtaaat 4260
   ataaaatttt taagtgtata atgtgttaaa ctactgattc taattgtttg tgtattttag 4320
   attccaacct atggaactga tgaatgggag cagtggtgga atgcctttaa tgaggaaaac 4380
   ctgttttgct cagaagaaat gccatctagt gatgatgagg ctactgctga ctctcaacat 4440
   tctactcctc caaaaaagaa gagaaaggta gaagacccca aggactttcc ttcagaattg 4500
   ctaagttttt tgagtcatgc tgtgtttagt aatagaactc ttgcttgctt tgctatttac 4560
   accacaaagg aaaaagctgc actgctatac aagaaaatta tggaaaaata ttctgtaacc 4620
   tttataagta ggcataacag ttataatcat aacatactgt tttttcttac tccacacagg 4680
   catagagtgt ctgctattaa taactatgct caaaaattgt gtacctttag ctttttaatt 4740
   tgtaaagggg ttaataagga atatttgatg tatagtgcct tgactagaga tcataatcag 4800
   ccataccaca tttgtagagg ttttacttgc tttaaaaaac ctcccacacc tccccctgaa 4860
   cctgaaacat aaaatgaatg caattgttgt tgttaacttg tttattgcag cttataatgg 4920
   ttacaaataa agcaatagca tcacaaattt cacaaataaa gcattttttt cactgcattc 4980
   tagttgtggt ttgtccaaac tcatcaatgt atcttatcat gtctggatca actggataac 5040
   tcaagctaac caaaatcatc ccaaacttcc caccccatac cctattacca ctgccaatta 5100
   cctagtggtt tcatttactc taaacctgtg attcctctga attattttca ttttaaagaa 5160
   attgtatttg ttaaatatgt actacaaact tagtagttgg aagggctaat tcactcccaa 5220
   agaagacaag atatccttga tctgtggatc taccacacac aaggctactt ccctgattag 5280
   cagaactaca caccagggcc aggggtcaga tatccactga cctttggatg gtgctacaag 5340
   ctagtaccag ttgagccaga taaggtagaa gaggccaata aaggagagaa caccagcttg 5400
   ttacaccctg tgagcctgca tgggatggat gacccggaga gagaagtgtt agagtggagg 5460
   tttgacagcc gcctagcatt tcatcacgtg gcccgagagc tgcatccgga gtacttcaag 5520
   aactgctgat atcgagcttg ctacaaggga ctttccgctg gggactttcc agggaggcgt 5580
   ggcctgggcg ggactgggga gtggcgagcc ctcagatcct gcatataagc agctgctttt 5640
   tgcctgtact gggtctctct ggttagacca gatctgagcc tgggagctct ctggctaact 5700
   agggaaccca ctgcttaagc ctcaataaag cttgccttga gtgcttcaag tagtgtgtgc 5760
   ccgtctgttg tgtgactctg gtaactagag atccctcaga cccttttagt cagtgtggaa 5820
   aatctctagc agtggcgccc gaacagggac ttgaaagcga aagggaaacc agaggagctc 5880
   tctcgacgca ggactcggct tgctgaagcg cgcacggcaa gaggcgaggg gcggcgactg 5940
   gtgagtacgc caaaaatttt gactagcgga ggctagaagg agagagatgg gtgcgagagc 6000
   gtcagtatta agcgggggag aattagatcg cgatgggaaa aaattcggtt aaggccaggg 6060
   ggaaagaaaa aatataaatt aaaacatata gtatgggcaa gcagggagct agaacgattc 6120
   gcagttaatc ctggcctgtt agaaacatca gaaggctgta gacaaatact gggacagcta 6180
   caaccatccc ttcagacagg atcagaagaa cttagatcat tatataatac agtagcaacc 6240
   ctctattgtg tgcatcaaag gatagagata aaagacacca aggaagcttt agacaagata 6300
   gaggaagagc aaaacaaaag taagaccacc gcacagcaag cggccgctga tcttcagacc 6360
   tggaggagga gatatgaggg acaattggag aagtgaatta tataaatata aagtagtaaa 6420
   aattgaacca ttaggagtag cacccaccaa ggcaaagaga agagtggtgc agagagaaaa 6480
   aagagcagtg ggaataggag ctttgttcct tgggttcttg ggagcagcag gaagcactat 6540
   gggcgcagcg tcaatgacgc tgacggtaca ggccagacaa ttattgtctg gtatagtgca 6600
   gcagcagaac aatttgctga gggctattga ggcgcaacag catctgttgc aactcacagt 6660
   ctggggcatc aagcagctcc aggcaagaat cctggctgtg gaaagatacc taaaggatca 6720
   acagctcctg gggatttggg gttgctctgg aaaactcatt tgcaccactg ctgtgccttg 6780
   gaatgctagt tggagtaata aatctctgga acagatttgg aatcacacga cctggatgga 6840
   gtgggacaga gaaattaaca attacacaag cttaatacac tccttaattg aagaatcgca 6900
   aaaccagcaa gaaaagaatg aacaagaatt attggaatta gataaatggg caagtttgtg 6960
   gaattggttt aacataacaa attggctgtg gtatataaaa ttattcataa tgatagtagg 7020
   aggcttggta ggtttaagaa tagtttttgc tgtactttct atagtgaata gagttaggca 7080
   gggatattca ccattatcgt ttcagaccca cctcccaacc ccgaggggac ccgacaggcc 7140
   cgaaggaata gaagaagaag gtggagagag agacagagac agatccattc gattagtgaa 7200
   cggatctcga cggtatcgat ggatctacaa atggcagtat tcatccacaa ttttaaaaga 7260
   aaagggggga ttggggggta cagtgcaggg gaaagaataa tagacataat agcaacagac 7320
   atacaaacta aagaattaca aaaacaaatt ataaaaattc aaaattttcg ggtttattac 7380
   agagacagca gagatccact ttgggatccc tcgagacgcg tgaattcccc ggggatcctc 7440
   tagagtcgac ctgcagaagc ttaccatggc cctgtggatg cgcctcctgc ccctgctggc 7500
   gctgctggcc ctctggggac ctgacccagc cgcagccttt gtgaaccaac acctgtgcgg 7560
   atccgacctg gtggaagctc tctacctagt gtgcggggaa cgaggcttct tctacacacc 7620
   caggaccaag cgggaggcag aggacctgca ggtggggcag gtggagctgg gcgggggccc 7680
   tggtgcaggc agcctgcagc ccttggccct ggagggatcc cggcagaagc gtggcattgt 7740
   ggaacaatgc tgtaccagca tctgctccct ctaccaactg gagaactact gcaactagtc 7800
   tagaggatcc ccggggaatt ccgccccccc cccctaacgt tactggccga agccgcttgg 7860
   aataaggccg gtgtgcgttt gtctatatgt tattttccac catattgccg tcttttggca 7920
   atgtgagggc ccggaaacct ggccctgtct tcttgacgag cattcctagg ggtctttccc 7980
   ctctcgccaa aggaatgcaa ggtctgttga atgtcgtgaa ggaagcagtt cctctggaag 8040
   cttcttgaag acaaacaacg tctgtagcga ccctttgcag gcagcggaac cccccacctg 8100
   gcgacaggtg cctctgcggc caaaagccac gtgtataaga tacacctgca aaggcggcac 8160
   aaccccagtg ccacgttgtg agttggatag ttgtggaaag agtcaaatgg ctctcctcaa 8220
   gcgtattcaa caaggggctg aaggatgccc agaaggtacc ccattgtatg ggatctgatc 8280
   tggggcctcg gtgcacatgc tttacatgtg tttagtcgag gttaaaaaac gtctaggccc 8340
   cccgaaccac ggggacgtgg ttttcctttg aaaaacacga tgataatatg gccacaacca 8400
   tggtgagcaa gggcgaggag ctgttcaccg gggtggtgcc catcctggtc gagctggacg 8460
   gcgacgtaaa cggccacaag ttcagcgtgt ccggcgaggg cgagggcgat gccacctacg 8520
   gcaagctgac cctgaagttc atctgcacca ccggcaagct gcccgtgccc tggcccaccc 8580
   tcgtgaccac cctgacctac ggcgtgcagt gcttcagccg ctaccccgac cacatgaagc 8640
   agcacgactt cttcaagtcc gccatgcccg aaggctacgt ccaggagcgc accatcttct 8700
   tcaaggacga cggcaactac aagacccgcg ccgaggtgaa gttcgagggc gacaccctgg 8760
   tgaaccgcat cgagctgaag ggcatcgact tcaaggagga cggcaacatc ctggggcaca 8820
   agctggagta caactacaac agccacaacg tctatatcat ggccgacaag cagaagaacg 8880
   gcatcaaggt gaacttcaag atccgccaca acatcgagga cggcagcgtg cagctcgccg 8940
   accactacca gcagaacacc cccatcggcg acggccccgt gctgctgccc gacaaccact 9000
   acctgagcac ccagtccgcc ctgagcaaag accccaacga gaagcgcgat cacatggtcc 9060
   tgctggagtt cgtgaccgcc gccgggatca ctctcggcat ggacgagctg tacaagtaat 9120
   gaattaatta agaattatca agcttatcga taccgtcgag acctggaaaa acatggagca 9180
   atcacaagta gcaatacagc agctaccaat gctgattgtg cctggctaga agcacaagag 9240
   gaggaggagg tgggtttcca gtcacacctc aggtaccttt aagaccaatg acttacaagg 9300
   cagctgtaga tcttagccac tttttaaaag aaaagggggg actggaaggg ctaattcact 9360
   cccaacgaag acaagatatc cttgatctgt ggattgaaag accccacctg taggtttggc 9420
   aagctagctt aagtaacgcc attttgcaag gcatggaaaa tacataactg agaatagaga 9480
   agttcagatc aaggttagga acagagagac agcagaatat gggccaaaca ggatatctgt 9540
   ggtaagcagt tcctgccccg gctcagggcc aagaacagat ggtccccaga tgcggtcccg 9600
   ccctcagcag tttctagaga accatcagat gtttccaggg tgccccaagg acctgaaaat 9660
   gaccctgtgc cttatttgaa ctaaccaatc agttcgcttc tcgcttctgt tcgcgcgctt 9720
   ctgctccccg agctcaataa aagagcccac aacccctcac tcggcgcgcc agtcctccga 9780
   tagactgcgt cgcccgggta cccgtagtgg cgagccctca gatgctgcat ataagcagct 9840
   gctttttgct tgtactgggt ctctctggtt agaccagatc tgagcctggg agctctctgg 9900
   ctaactaggg aacccactgc ttaagcctca ataagcttgc cttgagtgct tcactagaga 9960
   attcccnggg gatcctctag agtcgacctg cagaagctta ccatggccct gtggatgcgc 10020
   ctcctgcccc tgctggcgct gctggccctc tggggacctg acccagccgc agcctttgtg 10080
   aaccaacacc tgtgcggatc cgacctggtg gaagctctct acctagtgtg cggggaacga 10140
   ggcttcttct acacacccag gaccaagcgg gaggcagagg acctgcaggt ggggcaggtg 10200
   gagctgggcg ggggccctgg tgcaggcagc ctgcagccct tggccctgga gggatcccgg 10260
   cagaagcgtg gcattgtgga acaatgctgt accagcatct gctccctcta ccaactggag 10320
   aactactgca actagtctag aggatccccg gggaattctt gcagcccggg ggatccacta 10380
<210> 38
   <211> 20
   <212> DNA
   <213> Rattus sp.
<400> 38
   gactggttca acagccatgg 20
<210> 39
   <211> 20
   <212> DNA
   <213> Rattus sp.
<400> 39
   tagccgtcac agttgcagtc 20

## Description

### Field of the Invention

The present invention relates to the field of liver-directed nucleic acid sequence transfer. In particular the present invention relates to liver-directed gene therapy. The present invention also relates to the preparation of experimental animal models. The present invention also relates to pharmaceutical compositions comprising therapeutic viral vectors, particularly therapeutic lentiviral vectors and to surgical means of using same.

### Background

The liver represents an excellent organ for gene therapy since many genetic disorders result from deficiency of liver-specific gene products. In particular monogenic liver disorders or other disorders which may be treated by the provision of a gene product represent prime targets for the development of gene therapy-based treatments. To date, however, such approaches have suffered from a number of difficulties, including the lack of an efficient system for the delivery of specific nucleic acid sequences to the target cells.

One problem encountered in any gene therapy approach to the treatment of liver disease is that liver cells are not easy to transfect or transduce as they are quiescent non-dividing cells. Many vector systems, such as Moloney murine retroviral vectors, for example when used in research directed to therapeutic approaches for diabetes, lead to low levels of insulin production (Kolodka et al "Gene therapy for diabetes mellitus in rats by hepatic expression of insulin" Proc. Natl. Acad. Sci. USA 1995, 92:3293-3297) which are incapable of achieving a sustainable therapeutic effect.

There thus remains a need for improved methods of delivery for use in gene-based therapeutic approaches to liver diseases.

One disease which may benefit from improved treatment methods is Type I diabetes mellitus. Type I diabetes mellitus, also known as juvenile-onset diabetes or insulin-dependent diabetes mellitus, is caused by the autoimmune destruction of pancreatic beta cells that secrete insulin. Due to the absence of sufficient insulin, patients with type I diabetes have a reduced ability, or no ability, to regulate blood glucose levels, thereby leading to high blood glucose levels (hyperglycaemia). Over time, hyperglycaemia leads to the chronic complications of diabetes, including diabetic retinopathy, diabetic nephropathy, diabetic neuropathy and accelerated atherosclerosis. It is a life-long disease for which there is currently no cure.

Current treatment of the disease requires daily insulin injections to control blood glucose levels. Despite rigid maintenance and monitoring of blood glucose levels, the complications of diabetes still develop. Approximately 15% of people with type I diabetes die before age 40 years, the most common cause of death being diabetic ketoacidosis, kidney failure and heart disease.

As an alternative to daily insulin injections, transplantation of pancreatic tissue has been suggested. However, this is restricted by the scarcity of donors and requires patients to be maintained on long-term immunosuppression regimes.

Gene therapy approaches have also been suggested for the treatment of type I diabetes using liver cells. These approaches generally fall into one of two categories: approaches aimed at transformation of hepatocytes into insulin-producing pancreatic tissue and approaches which aim to induce insulin expression in liver cells without inducing pancreatic transdifferentiation. Each approach suffers from a number of disadvantages. For example, in the latter, research into therapies based on expression of insulin in cells transfected with recombinant insulin have been hampered by low transduction efficiency.

One problem associated with previous cell-based therapies in which liver cells are transfected or transduced to express insulin is the inability of the cells to store insulin. Without this ability, insulin may be produced in an unregulated manner and recipients containing the recombinant cells may become hypoglycaemic and die.

In an attempt to address this problem several studies have examined transcriptional control of insulin expression without induction of storage (Lu et al., "Regulatable production of insulin from primary cultured hepatocytes: insulin production is up-regulated by glucagon and cAMP and down-regulated by insulin" Gene Therapy 1998, 5:888-95; Thule et al., "Glucose regulated production of human insulin in rat hepatocytes" Gene Therapy 2000, 7:205-14). Transcription control, however, does not control insulin secretion and there is an inevitable delay in regulation of blood glucose levels with concomitant therapeutic deficiencies. Furthermore, when contemplated for use in such a system, the liver-specific glucose-sensitive promoters have elements that respond to hormonal and metabolic signals that may impede, attenuate or abrogate the desired objective of tight glucose regulation.

An alternative approach that has been taken to the storage problem is to induce liver to pancreas transdifferentiation (Ber et al "Functional, persistent, and extended liver to pancreas transdifferentiation" J. Biol. Chem. 2003, 278:31950-7; Kojima et al "NeuroD-betacellulin gene therapy induces islet neogenesis in the liver and reverses diabetes in mice" Nat. Med. 2003, 9:596-603). To date these methods have shown limited potential for therapeutic application.

Numerous mammalian liver diseases and conditions associated with the absence of a functional gene product or the presence of an abnormal gene product are known. Such conditions include, but are not limited to, familial hypercholesterolemia which is an autosomal dominant disorder due to a mutation in the low density lipoprotein (LDLR) gene, hemophilia which is associated with a lack of Factor VIII (hemophilia A) or a lack of Factor IX (hemophilia B), alpha-1-antitrypsin deficiency, Criglar Najar syndrome which is associated with an abnormality of hepatic bilirubin UDP-glucuronyltransferase. For a number of these conditions attempts at therapeutic methods involving gene therapy have been reported. Although promising results have on occasion been reported in some limited circumstances, there is at present no suitable method for the efficacious treatment of such diseases using gene therapy.

There thus remains a need for an improved method for the treatment of disorders such as type I diabetes mellitus and others which may benefit from improved liver-directed nucleic acid transfer.

### Summary of the Invention

In a first aspect of the invention there is provided a method of introducing a nucleic acid molecule into a liver cell of a mammal, the method comprising
(i) inhibiting blood flow to the liver of said mammal;
(ii) transfusing liver cells of said mammal with a lentiviral vector, the vector comprising said nucleic acid molecule;
(iii) restoring blood flow to and from said liver.

In one embodiment, inhibition of blood flow to the liver may comprise substantially complete inhibition of blood flow.

In one embodiment, inhibition of blood flow to the liver results in the liver being substantially drained of blood.

In one embodiment, inhibition of blood flow to the liver may comprise ligating or clamping the right adrenal vein of said liver, successively clamping the infrahepatic vena cava, the hepatic artery, the portal vein, and the suprahepatic vena cava of said mammal.

Inhibition of blood flow to and from the liver may be for any suitable period of time, such as any period of time between about one minute and about ten minutes. For example, inhibition of the blood flow may be for about 1 minute, about 2 minutes, about 3 minutes, about 4 minutes, about 5 minutes, about 6 minutes, about 7 minutes, about 8 minutes, about 9 minutes, or about 10 minutes. In one embodiment inhibition of blood flow is for about 5 minutes.

In one embodiment, transfusing liver cells comprises portal infusion, such as through a cannula inserted in the portal vein.

In one embodiment, the lentiviral vector may be selected from the group consisting of HIV-MSCV hybrid viral vectors.

The lentiviral vector may be used in any suitable amount. In one embodiment, between at least about 0.5x10⁵ and at least about 8x10⁶ transduction units of the lentiviral vector may be administered.

In one embodiment the nucleic acid molecule may be selected from the group consisting of nucleic acid sequences encoding a polypeptide having biological activity of insulin, factor VIII, factor IX, alpha 1 antitrypsin, hepatic bilirubin UDP-glucuronyltransferase or low density lipoprotein receptor, or a precursor thereof.

In one embodiment, the nucleic acid molecule comprises, or is in operable association with a promoter and/or enhancer sequence.

In one embodiment, restoring blood flow to the liver may be permanent or temporary.

Temporary restoration of blood flow to the liver may be for any suitable time to allow liver cells to be bathed in blood, such as any period of time between about 1 minute and about 5 minutes. For example, the period of time may be about 1 minute, or about 2 minutes, or about 3 minutes, or about 4 minutes, or about 5 minutes. In one embodiment, the suitable time is any period of time between about 2 minutes and about 3 minutes.

In one embodiment, restoration of blood flow to the liver comprises successively restoring blood flow to the suprahepatic vena cava, the infrahepatic vena cava, the portal vein and the hepatic artery. Optionally, the right adrenal vein may be released.

The steps (i), (ii) and (iii) may be repeated. For example, the steps may be repeated one, two, three, or four times. In one embodiment, the steps are repeated two times.

The vector may comprise additional nucleic acid sequences, such as secretion tags, inducible promoters/enhancers, such as hepatocyte-specific promoters.

In one embodiment of the first aspect the method comprises:
(i) ligating or clamping the right adrenal vein of the liver of said mammal;
(ii) successively clamping the infrahepatic vena cava, the hepatic artery, the portal vein, and the suprahepatic vena cava of said mammal;
(iii) transfusing liver cells of said mammal with a lentiviral vector, said transfusion comprising infusion through the portal vein of said mammal, the vector comprising said nucleic acid molecule;
(iv) restoring blood flow to the liver, the restoration comprising successively restoring blood flow to the suprahepatic vena cava, optionally releasing the right adrenal vein, and restoring blood flow to the infrahepatic vena cava, the portal vein and the hepatic artery;
(v) optionally repeating steps (ii) to (iv) one, two or three times.

In one embodiment, the nucleic acid molecule comprises, or is in operable association with a promoter and/or enhancer sequence.

In a second aspect of the invention there is provided a method for the treatment of type I diabetes mellitus in a mammal, the method comprising:
(i) inhibiting blood flow to the liver of said mammal;
(ii) transfusing liver cells of said mammal with a lentiviral vector, the vector comprising a nucleic acid sequence encoding a polypeptide having biological activity of insulin or a precursor polypeptide thereof;
(iii) restoring blood flow to and from said liver.

In one embodiment, inhibition of blood flow to the liver may comprise substantially complete inhibition of blood flow.

In one embodiment, inhibition of blood flow to the liver results in the liver being substantially drained of blood.

In one embodiment, inhibition of blood flow to the liver may comprise ligating or clamping the right adrenal vein of said liver, successively clamping the infrahepatic vena cava, the hepatic artery, the portal vein, and the suprahepatic vena cava of said mammal.

Inhibition of blood flow to and from the liver may be for any suitable period of time, such as any period of time between about one minute and about ten minutes. For example, inhibition of the blood flow may be for about 1 minute, about 2 minutes, about 3 minutes, about 4 minutes, about 5 minutes; about 6 minutes, about 7 minutes, about 8 minutes, about 9 minutes, or about 10 minutes. In one embodiment inhibition of blood flow is for about 5 minutes.

In one embodiment, transfusing liver cells comprises portal infusion, such as through a cannula inserted in the portal vein.

In one embodiment, the lentiviral vector may be selected from the group consisting of HIV-MSCV hybrid viral vectors.

In one embodiment the nucleic acid encodes a furin-cleavable insulin or proinsulin sequence, such as human proinsulin.

In one embodiment, the nucleic acid comprises, or is in operable association with a promoter and/or enhancer sequence.

In one embodiment the vector is the lentiviral vector MSCV/HIV-INS-FUR.

The lentiviral vector may be used in any suitable amount. In one embodiment, between at least about 0.5x10⁵ and at least about 8x10⁶ transduction units of the lentiviral vector may be administered.

In one embodiment, restoring blood flow to the liver may be permanent or temporary.

Temporary restoration of blood flow to the liver may be for any suitable time to allow liver cells to be bathed in blood, such as any period of time between about 1 minute and about 5 minutes. For example, the period of time may be about 1 minute, or about 2 minutes, or about 3 minutes, or about 4 minutes, or about 5 minutes. In one embodiment, the suitable time is any period of time between about 2 minutes and about 3 minutes.

In one embodiment, restoration of blood flow to the liver comprises successively restoring blood flow to the suprahepatic vena cava, the infrahepatic vena cava, the portal vein and the hepatic artery. Optionally, the right adrenal vein may be released.

The steps (i), (ii) and (iii) may be repeated. For example, the steps may be repeated one, two, three, or four times. In one embodiment, the steps are repeated two times.

In one embodiment of the second aspect the method comprises:
(i) ligating or clamping the right adrenal vein of the liver of said mammal;
(ii) successively clamping the infrahepatic vena cava, the hepatic artery, the portal vein, and the suprahepatic vena cava of said mammal;
(iii) transfusing liver cells of said mammal with a lentiviral vector, said transfusion comprising infusion through the portal vein of said mammal, the vector comprising a furin-cleavable insulin gene;
(iv) restoring blood flow to the liver, the restoration comprising successively restoring blood flow to the suprahepatic vena cava, optionally releasing the right adrenal vein, and restoring blood flow to the infrahepatic vena cava, the portal vein and the hepatic artery;
(v) optionally repeating steps (ii) to (iv) one, two or three times.

In a third aspect of the invention there is provided a method for the preparation of a mammal capable of expressing a transgene in a liver cell, the method comprising:
(i) inhibiting blood flow to the liver of said mammal;
(ii) transfusing liver cells of said mammal with a lentiviral vector, the vector comprising a nucleic acid molecule comprising said transgene;
(iii) restoring blood flow to and from said liver.

In one embodiment the nucleic acid encoding said transgene is selected from nucleic acid molecules encoding a polypeptide having biological activity of insulin, factor VIII, factor IX, alpha 1 antitrypsin, hepatic bilirubin UDP-glucuronyltransferase or low density lipoprotein receptor, or a precursor thereof.,

In a fourth aspect of the invention there is provided a non-human mammal capable of expressing a transgene in a liver cell prepared by the method of the third aspect.

In further aspects of the invention there is also provided use of a lentiviral vector for introducing a nucleic acid molecule into a liver cell of a mammal according to the methods of the invention, use of a lentiviral vector for introducing a furin-cleavable insulin gene into a liver cell of a mammal having type I diabetes mellitus according to the methods of the invention, and pharmaceutical compositions of lentiviral vectors suitable for use in the methods of the invention.

In a further aspect of the invention there is provided a method for the isolation of hepatocytes from an *in vivo* source, the method comprising dual perfusion of the portal vein and the abdominal aorta.

In a further aspect of the invention there is provided an hepatocyte culture medium comprising DMEM supplemented with NaHCO₃, insulin, glutamic acid, proline, ascorbic acid, penicillin, nicotinamide, epidermal growth factor, selenium salts and hydrocortisone modified DMEM.

In a preferred embodiment the hepatocyte culture medium comprises about 44 mmol/L NaHCO₃, about 100 mU/L insulin, about 2.5 mmol/L glutamic acid, about 1 mmol/L proline, about 56 mg/L ascorbic acid, about 100 mg/ml penicillin, about 10 mmol/L nicotinamide, about 20 ng/ml epidermal growth factor, 0.1 µg/ml selenium salts and about 1 mnol/L hydrocortisone.

### Abbreviations

STZ: streptozotocin.
AAT: alpha 1 antitrypsin.
HSV: herpes simplex virus.
MSCV: murine stem cell virus.
HIV: human immunodeficiency virus.
LTR: long terminal repeat.
EGFP: enhanced green fluorescent protein.
NOD mice: nonobese diabetic mice.
PV: portal vein.
AA: abdominal aorta.
DMEM: Dulbecco's minimal essential medium.

### Definitions

The following are some definitions that may be helpful in understanding the description of the present invention. These are intended as general definitions and should in no way limit the scope of the present invention to those terms alone, but are put forth for a better understanding of the following description.

In the context of this specification, gene therapy or gene-based therapy refers to the treatment or prevention of a disease by introduction of genetic material, often engineered, into an organism, tissue or cell in order to alter some function within that organism, tissue or cell.

In the context of this specification, the term "transduction", and variants thereof such as "transduce", refers to the transfer of genetic material into liver cells of a recipient mammal.

In the context of this specification, the term "transfusion" refers to the introduction of a fluid into a blood vessel of a recipient mammal and is used interchangeably with the term "infusion".

In the context of this specification, the term "polypeptide" means proteins, peptides, protein fragments, modified proteins, amino acid sequences and synthetic amino acid sequences. The polypeptide can be post-translationally modified, such as glycosylated, or not.

In the context of this specification, the term "regulatory sequence" refers to any sequences that influence expression of an operably associated nucleic acid sequence, such as a gene sequence, once the vector is integrated in the target cell as a provirus. Thus, the term "regulatory sequence" includes sequences which may be upstream or downstream of the operably associated nucleic acid sequence, for example, promoter sequences, enhancer sequences, glucose-regulatable promoter sequences, hepatocyte-specific promoter sequences.

In the context of this specification, the term "furin" refers to the subtilisin-like eukaryotic endopeptidase with substrate specificity for consensus sequence Arg-X-Lys/Arg-Arg, and variants thereof.

In the context of this specification, the term "insulin" will be understood to include insulin and variants thereof. Likewise the terms "proinsulin" and "preproinsulin" will be understood to include variants thereof.

In the context of this specification, the term "treatment" refers to any and all uses which remedy a disease state or symptoms, prevent the establishment of disease, or otherwise prevent, hinder, retard, or reverse the progression of disease or other undesirable symptoms in any way whatsoever.

The term "therapeutically effective amount" as used herein, includes within its meaning a non-toxic but sufficient amount a compound or composition for use in the invention to provide the desired therapeutic effect. The exact amount required will vary from subject to subject depending on factors such as the species being treated, the age and general condition of the subject, the severity of the condition being treated, the particular agent being administered and the mode of administration and so forth. Thus, it is not possible to specify an exact "effective amount". However, for any given case, an appropriate "effective amount" may be determined by one of ordinary skill in the art using only routine experimentation.

In the context of this specification, the term "expression" refers interchangeably to expression of a gene or gene product, including the encoded protein. Expression of a gene may be determined, for example, by measuring the production of messenger RNA (mRNA) transcript levels. Expression of a polypeptide gene product may be determined, for example, by immunoassay using an antibody(ies) that bind with the polypeptide.

In the context of this specification, the term "ligating" a vessel such as a blood vessel, refers to permanent constriction or closure of the vessel.

In the context of this specification, the term "clamping" a vessel such as a blood vessel, refers to a temporary or semi-permanent constriction or closure of the vessel, such that removal of the clamp is associated with partial or complete restoration of flow through the vessel.

In the context of this specification, the term "mammal" includes humans and individuals of any mammalian species of social, economic or research importance including but not limited to members of the genus ovine, bovine, equine, porcine, feline, canine, primates, rodents.

In the context of this specification, MSCV/HIV-INS-FUR and HMD/INS-FUR refer to the same vector construct.

In the context of this specification, the term "about" when used in respect of amounts or values, will be understood to indicate that the stated amount includes a variation of plus or minus 50% of the stated amount or value.

Unless the context requires otherwise or specifically stated to the contrary, integers, steps, or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of elements or integers. Thus, in the context of this specification, the term "comprising" means "including principally, but not necessarily solely".

Those skilled in the art will appreciate that the invention described herein is susceptible to variations and modifications other than those specifically described. It is to be understood that the invention includes all such variations and modifications. The invention also includes all of the steps, features, compositions and compounds referred to or indicated in this specification, individually or collectively, and any and all combinations or any two or more of said steps or features.

Any discussion of documents, acts, materials, devices, articles or the like which has been included in the present specification is solely for the purpose of providing a context for the present invention. It is not to be taken as an admission that any or all of these matters form part of the prior art base or were common general knowledge in the field relevant to the present invention before the priority date of this application. For the purposes of description all documents referred to herein are incorporated by reference.

### Brief Description of the Figures

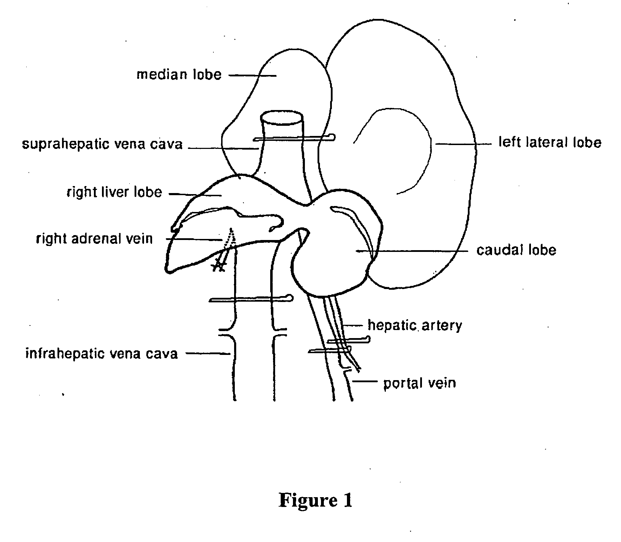

**Figure 1****:** Schematic diagram of asanguineous perfusion via the portal vein.

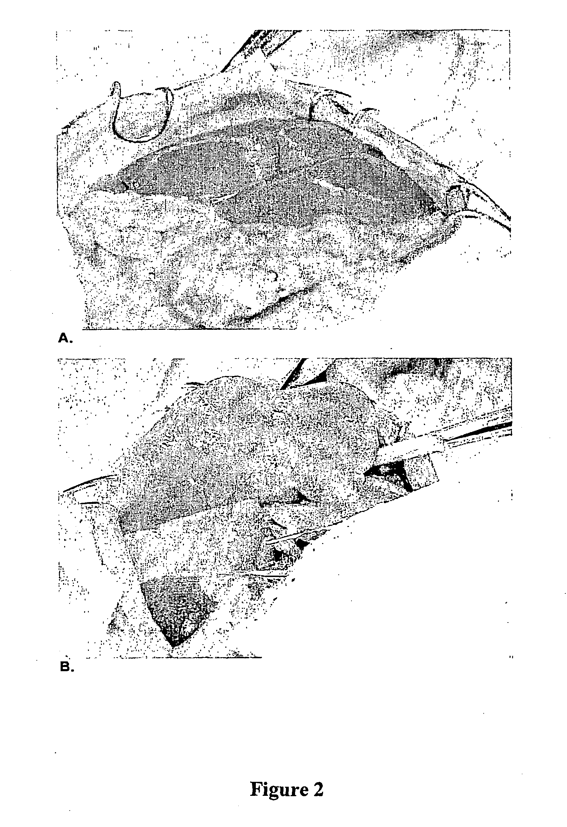

**Figure 2****:** Photographs of **(A)** normal liver tissue prior to surgery and **(B)** liver ligated, clamped and drained of blood prior to vector infusion.

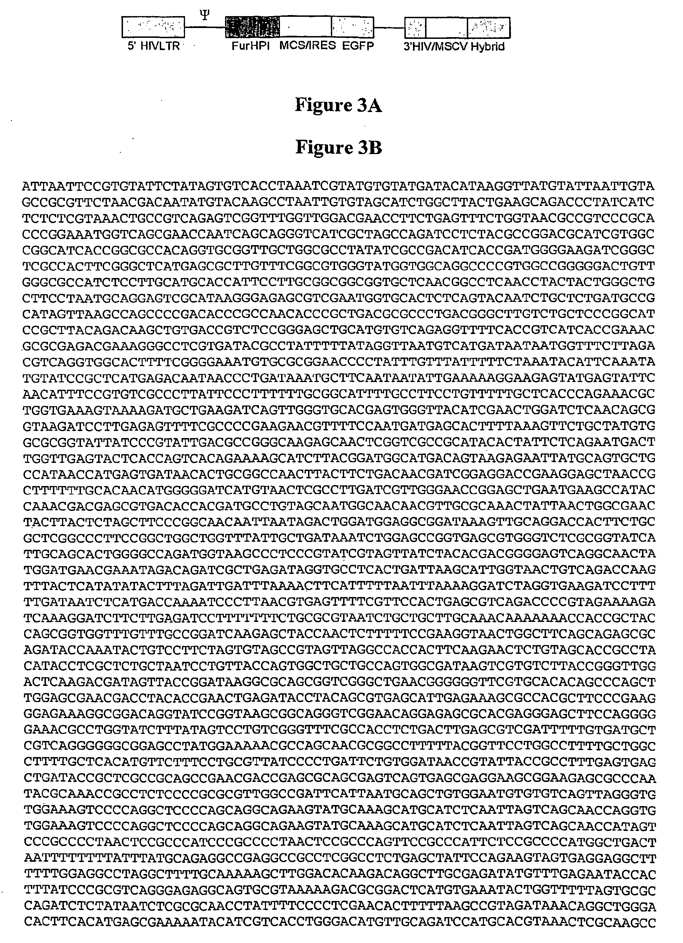

**Figure 3A****:** Schematic illustration of the lentiviral vector HMD/INS-FUR containing the furin-cleavable form of human proinsulin cDNA (FurHPI). The insulin cassette was cloned into the multiple cloning site MCS of the vector. HIV/MSCV represents the portion of the 3' HIVLTR and murine stem cell virus (MCSV) hybrid enhancer/promoter. LTR: long terminal repeats. The viral packaging signal is indicated by Ψ. IRES: internal ribosomal entry site. EGFP: enhanced green fluorescent protein.

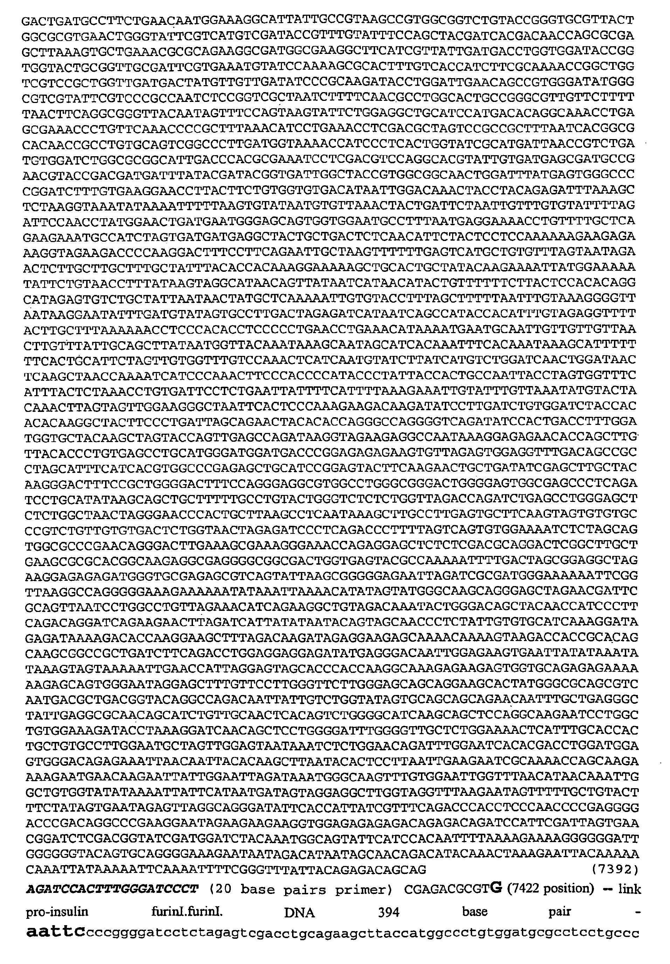

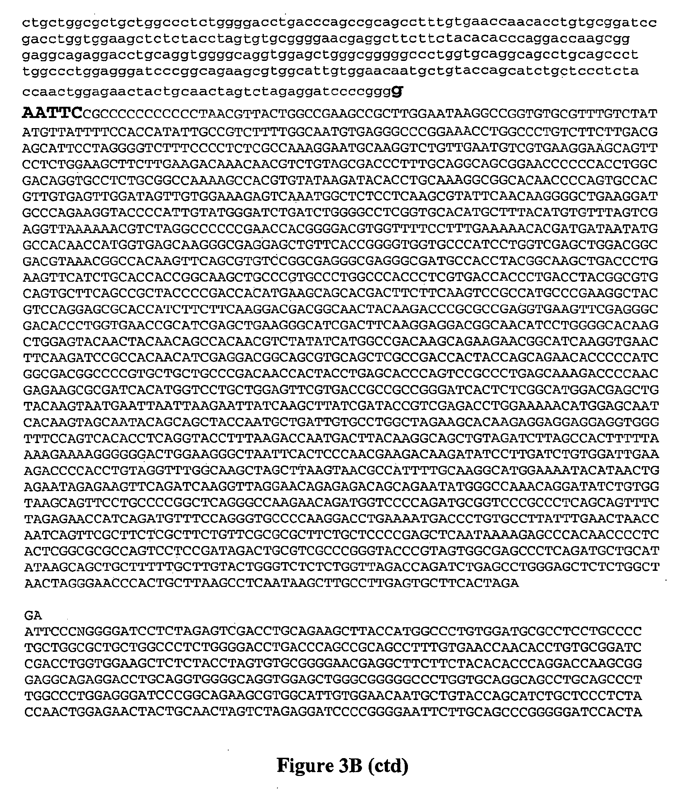

**Figure 3B****: Nucleotide sequence of HMD vector with Furin-modified insulin.** (SEQ ID NO:37). Proinsulin furinI.furinII.BIOD was isolated from the pRIS.proinsulin.Ifur.Iifur.BIOD cloning vector (Genentech) by restriction digest at two EcoR I sites, one upstream of the 5' transcription start site and one downstream of the 3' terminal signal of the proinsulin gene. The proinsulin gene fragment was 394 base pairs. The 394 base pair proinsulin was subsequently inserted into the HMD vector at 7422 DNA site position (Figure 3). The sequencing primer for HMD vector was designed from position 7392 to 7411 base pairs (high light section). DNA sequencing data identified that pro-insulin furinI.furinII DNA is in the HMD vector 7422 base pair position (Figure 3b, lower case).

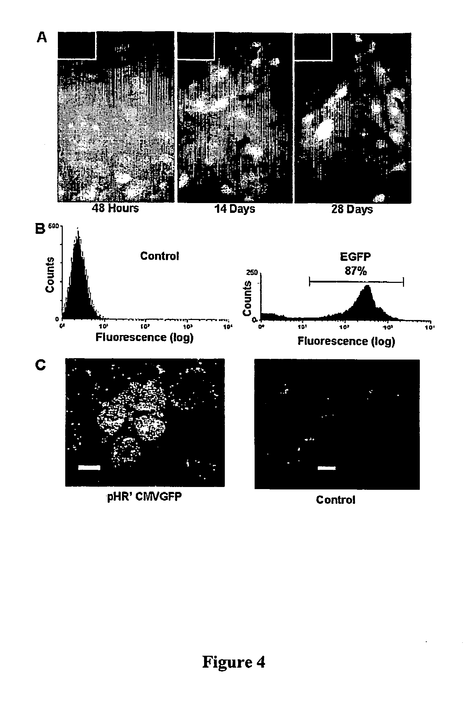

**Figure 4****: EGFP expression in primary hepatocytes *in vitro* and *in vivo.* (a)** EGFP expression in primary hepatocytes *in* vitro 48 hr, 14 and 28 days post transduction with pHR'CMVGFP. Original magnification 400X. Inset represents untransduced hepatocytes at each time point. **(b)** Flow cytometric analysis of irradiated non-dividing primary rat hepatocytes transduced with pHR'CMVGFP *in* vitro. The x-axis shows the intensity of EGFP fluorescence and the y-axis the cell number. Negative control cells show background fluorescence only. EGFP-positive cells constituted 87% of all the cells in the experiment following transduction. Representative of 3 independent experiments. (**c**) Confocal images of EGFP expression recorded from primary rat hepatocytes isolated by collagenase perfusion from the liver of an animal transduced by the lentiviral vector pHR'CMVGFP and saline (control), 6 months after initial transduction. It is obvious from this figure that stable expression of EGFP is visible in a large proportion of cells while there is a small amount of autofluorescence present in the control. Bar = 20 µm.

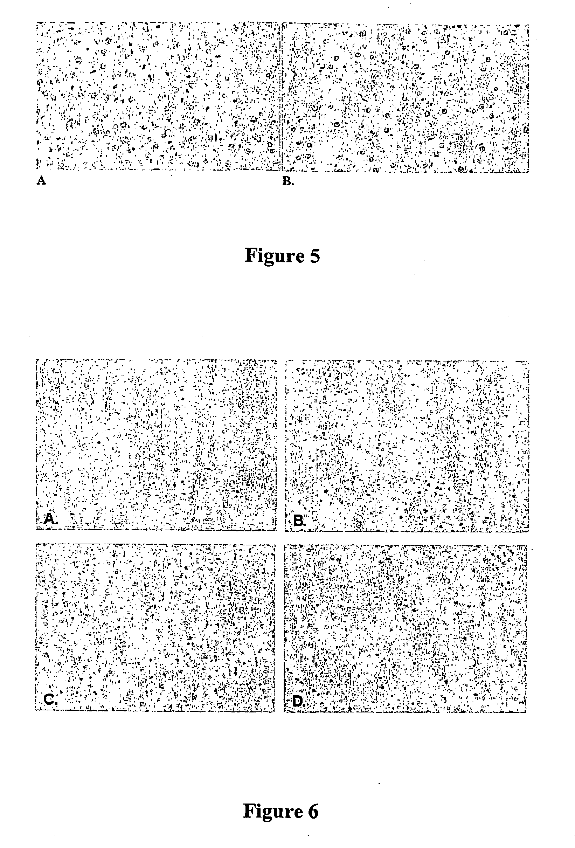

**Figure 5****:** Photomicrographs of (**A**) a section of the liver of a STZ-diabetic Wistar rat transduced with the viral vector alone for a period of 2 months; (**B**) a section of the liver of a Wistar rat transduced with the viral vector containing MSCV/ HIV-INS-FUR for a period of 2 months. Immunoperoxidase for insulin, positively stained cells are brown. 200X Magnification
**Figure 6****:** Photomicrographs of (**A**) A section of the liver of a STZ-diabetic Wistar rat transduced with the lentiviral vector (MSCV)/ HIV alone for a period of 2 months, (**B**) A section of the liver of a Wistar rat transduced with the lentiviral vector MSCV/ HIV-INS-FUR for a period of 2 months. Immunoperoxidase for glucagon, positively stained cells are brown. (**C**) A section of the liver of a STZ-diabetic Wistar rat transduced with the lentiviral vector (MSCV)/ HIV alone for a period of 2 months, (**D**) A section of the liver of a Wistar rat transduced with the lentiviral vector MSCV/ HIV-INS-FUR for a period of 2 months. Immunoperoxidase for somatostatin, positively stained cells are brown. 200X Magnification.

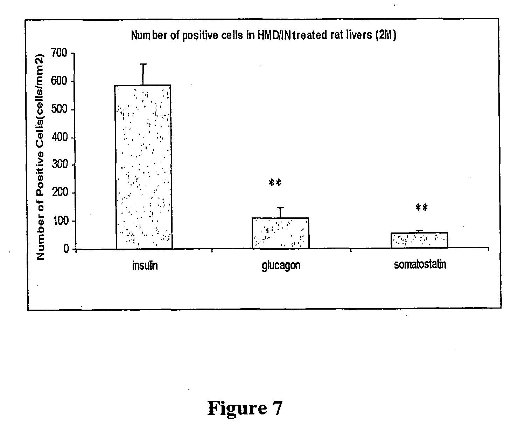

**Figure 7****:** Summary of immunostaining analysis. The livers of diabetic rats treated with HMD/INS- FUR vector were subjected to immunostaining 2 months after injection. Insulin positive cells were 585.6/mm², 4.8-fold higher than glucagon (109.8/mm²) and 11.2-fold higher than somatostatin (52.2/ mm²). Results expressed as mean ± SD (n=3).

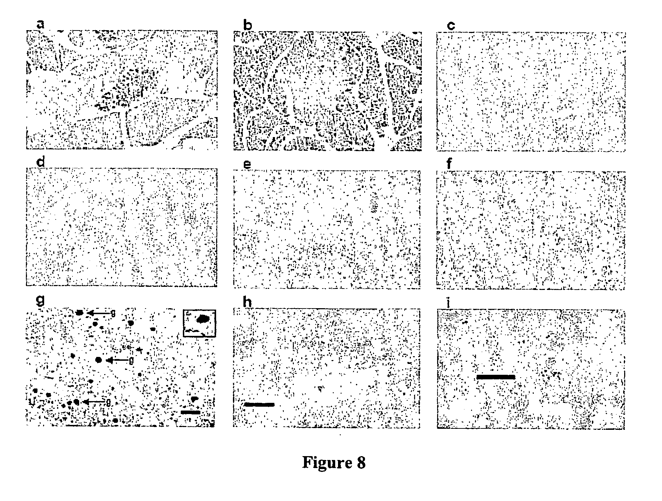

**Figure 8****: Expression of pancreatic hormones following correction STZ-induced hyperglycemia in rat livers**. Photomicrographs of anti-insulin staining of (**a**) a normal control pancreas; **(b)** STZ-diabetic HMD/INS-FUR vector-treated pancreas; (**c**) normal control rat liver, and **(d)**. STZ-diabetic HMD/INS-FUR vector-treated liver at 2 months. Photomicrographs of anti-glucagon **(e)** and anti-somatostatin staining **(f)** in STZ-diabetic HMD/INS-FUR vector-treated rat liver at 2 months; positively stained cells are brown (Magnification: x200). Control liver sections stained for glucagon and somatostatin were negative (not shown). Cells staining positive for pancreatic hormones were distributed evenly around the liver tissue in animals, which had undergone FFO, not isolated to the regions around the portal circulation. These results were not significantly different in animals maintained for 500 days. Transmission electron micrograph **(g)** showing secretory vesicles with dense granules (g) of approximately 300-400 nm in diameter surrounded by a pale halo, Bar = 500 nm. Inset shows a vesicle of 320 nm in diameter. **(h)** Immuno-electron micrographs showing localization of insulin at 2 months in STZ-diabetic HMD/INS-FUR vector-treated liver, bar = 400 nm and in Nit-1 cells **(i)** (positive control), bar = 400 nm. The above results were consistent with results at 500 days.

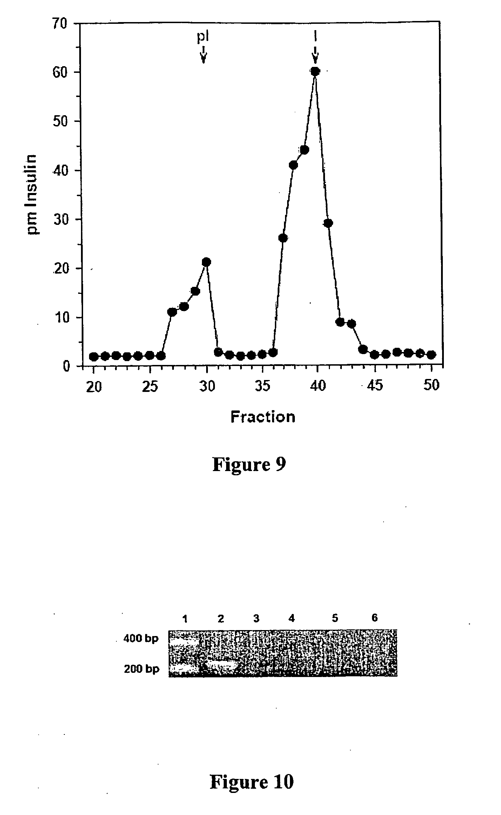

**Figure 9****: Stable expression of INS-FUR in rat livers results in production of mature human insulin**. Acid ethanol extracts of rat livers were separated on a Sephadex G-50 super fine column and were collected and assayed for insulin content. Mobility of mature human insulin (i) (Mol. Wt = 5,800) and proinsulin (pI) (Mol. Wt. = 9, 400) are indicated by arrows. Representative of 3 independent experiments
**Figure 10****: RT - PCR for the presence of INS-FUR**. Total RNA was extracted from liver, pancreas, spleen and kidney of rats 2 months after transduction with HMD/INS-FUR Lane 1: DNA marker, lane 2: liver, lane 3: pancreas, lane 4: spleen, lane 5: kidney.

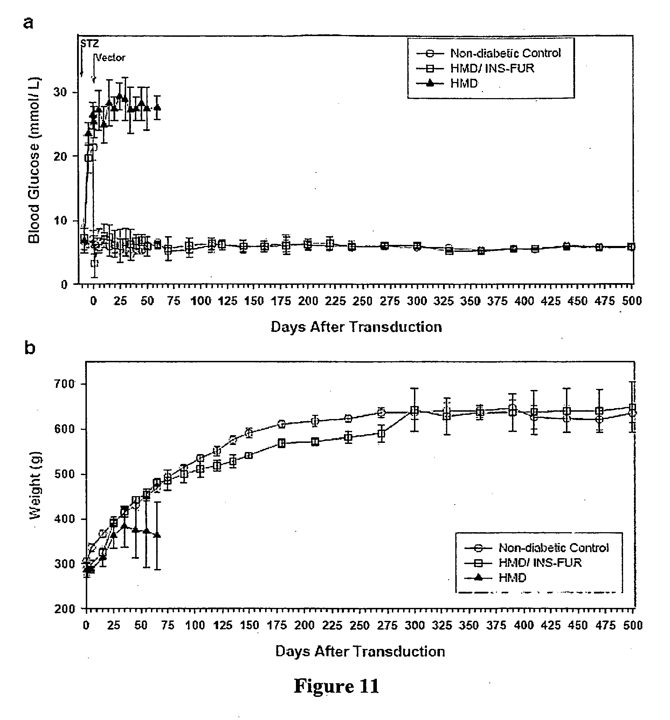

**Figure 11****: Stable expression of INS-FUR in rat livers ameliorates STZ-induced hyperglycemia (a)** Blood glucose of STZ-diabetic Wistar rats treated with HMD/ INS-FUR or HMD alone. (n=5). (b) Body weights *of* STZ-diabetic Wistar rats treated with HMD/ INS-FUR or HMD alone. (n=5).

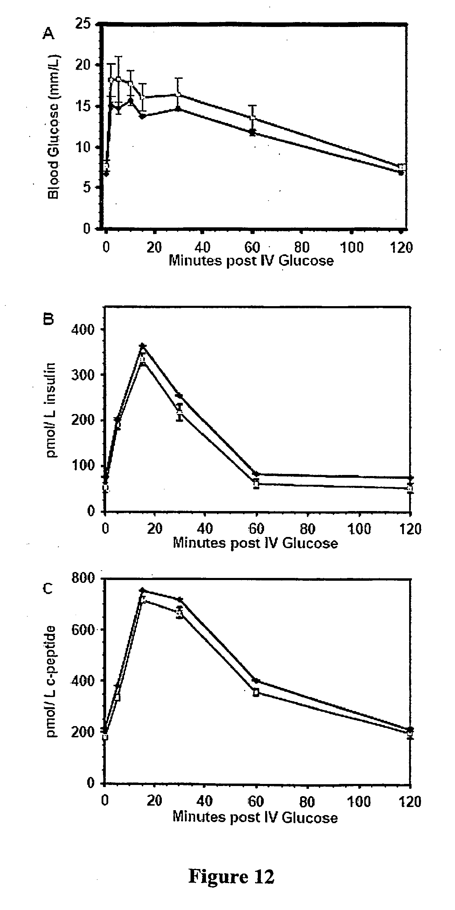

**Figure 12****: (a) Plasma glucose, (b) insulin and** (**c**) **c-peptide levels after an IVGTT of STZ-diabetic HMD/INS-FUR vector-treated rats. (n=5)**. 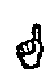 : HMD/ INS-FUR treated animals (express human insulin and c-peptide); γ: normal control animals (express rat insulin and c-peptide).

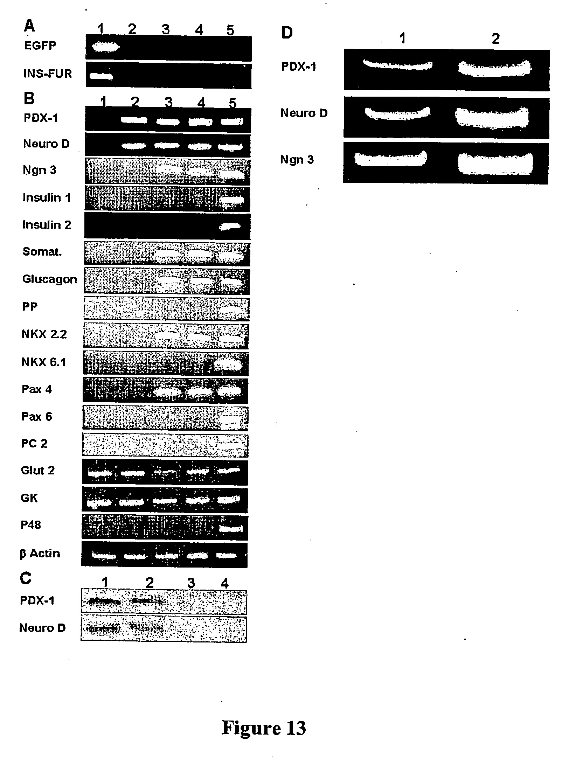

**Figure 13****: β-cell transcription factors and pancreatic hormones expressed in transduced rat livers. (a)** RT-PCR analysis of rat tissues for EGFP or INS-FUR in animals transduced with the pHR'CMVGFP or the HMD/ INS-FUR lentiviral vectors respectively: liver (lane 1), pancreas (lane 2), spleen (lane 3), kidney (lane 4) and brain (lane 5) and **(b)** RT-PCR analysis for various β-cell transcription factors, pancreatic endocrine hormones: somatostatin (somat.), glucagon and pancreatic polypeptide (PP); GLUT 2 and glucokinase (GK); rat insulin 1 and 2; insulin proconvertase PC2; exocrine marker p48 and β-actin in normal rat liver (lane 1), rat liver transduced with HMD alone at 60 days (lane 2), rat liver transduced with the HMD/ INS-FUR lentiviral vectors at 60 days (lane 3) and 500 days (lane 4) and normal rat pancreas (lane 5). (c) Qualitative Western blot analysis for PDX-1, NeuroD in normal rat pancreas (lane 1), rat liver transduced with the HMD/ INS-FUR lentiviral vectors at 60 days (lane 2), rat liver transduced with HMD alone at 60 days (lane 3), and normal rat liver (lane 4). PDX-1 and NeuroD have bands at 42 and 50 kD respectively. (d) Real time PCR analysis of PDX-1, NeuroD and Ngn3 in rat liver transduced with the HMD/ INS-FUR lentiviral vectors at 500 days (lane 1) and normal rat pancreas (lane 2).

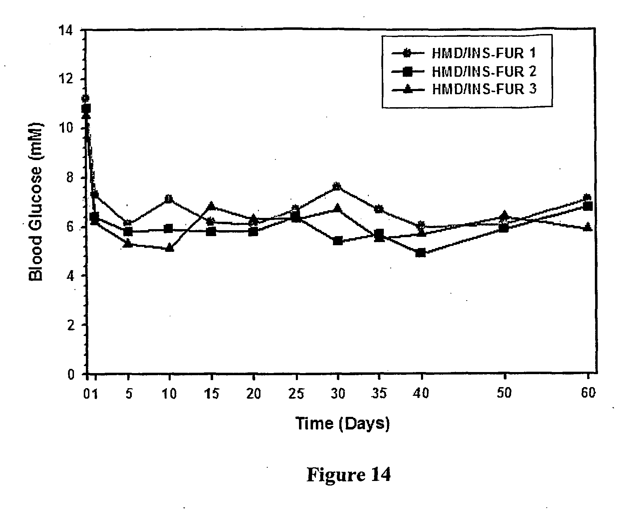

**Figure 14****:** Serum glucose of NOD mice treated with the HMD/INS-FUR vector. Blood glucose levels fell rapidly to normal levels on addition of the HMD/INS-FUR vector and normoglycaemia was maintained for 2 months when the animals were sacrificed. Mice treated with the vector alone or untreated remained diabetic until sacrifice (results not shown).

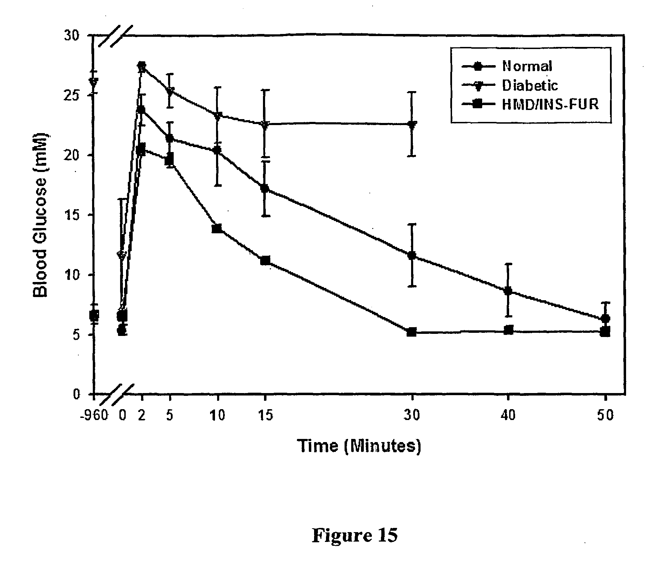

**Figure 15****:** Intravenous glucose tolerance test: after an overnight fast on non diabetic control NOD mice, diabetic NOD mice and diabetic NOD mice treated with HMD/INS-FUR vector 2 months previously. The animals were given an intravenous dose of glucose (0.5 g/ kg body weight), blood glucose levels were measured over 30-50 min (n=3).

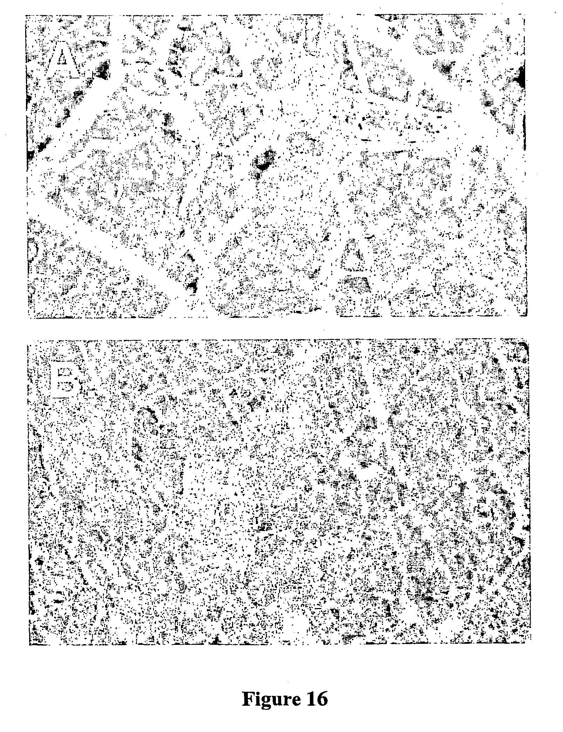

**Figure 16****:** Photomicrographs of **(A)** A section of the pancreas of an NOD mouse before the onset of diabetes as judged by blood glucose level; **(B)** A section of the pancreas of a diabetic NOD mouse. After immunohistochemistry for insulin using immunoperoxidase, positive cells appeared brown. Original magnification = 200 X.

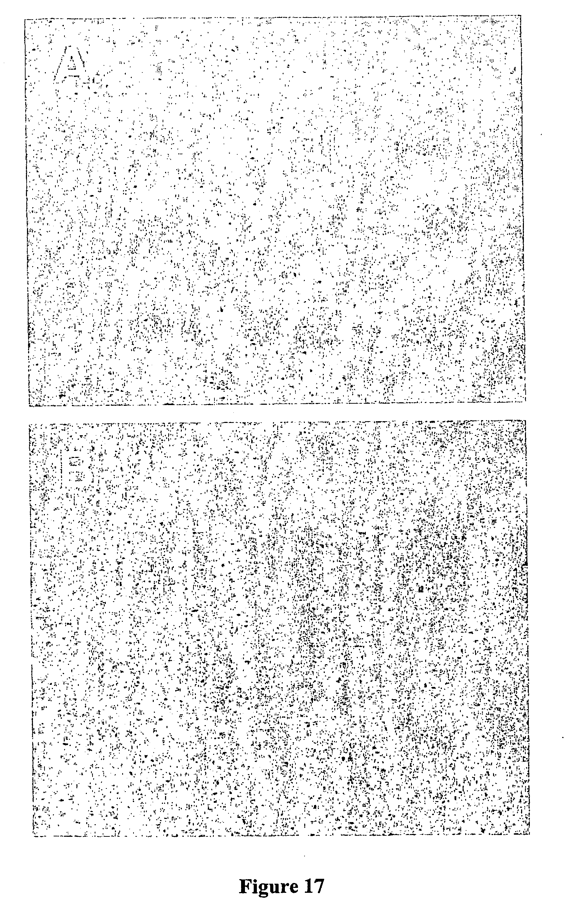

**Figure 17****:** Photomicrographs of **(A)** A section of the liver of an untransduced NOD mouse; **(B)** A section of the liver of an NOD mouse transduced with the HMD/INS-FUR vector for a period of 2 months. After immunohistochemistry for insulin using immunoperoxidase, positive cells appeared brown. Original magnification = 200 X.

### Detailed Description and Preferred Embodiments

The liver represents an excellent organ for gene therapy since many genetic disorders result from deficiency of liver-specific gene products. As described herein the present invention provides an effective method for the introduction of a transgene into liver cells and the subsequent long term expression of the trans gene-encoded product. As described in the Examples herein the efficacy of the invention is demonstrated using type I diabetes mellitus as an example system, by long term stable reversal of STZ-diabetes in Wistar rats. This demonstration of efficacy of the invention in treatment of diabetes should not be construed as limiting the invention in any way. As the skilled addressee will appreciate the invention may be used in diverse situations involving the introduction of a gene or nucleic acid sequence into a liver cell, such as for the treatment of diverse conditions and diseases. Diseases that may be treated using the methods of the invention include all forms of gene defects, including single gene defects, where replacement of a defective gene, or delivery of a gene to the liver would alleviate or cure the disease, condition, or symptoms thereof. It is also anticipated that diseases that may be treated by the method of the invention include diseases and conditions susceptible to genetic prodrug activation therapy, such as hepatic cancer. It is also anticipated that recipient transgenic mammals may be useful as experimental animals, such as for investigation of disease conditions and potential therapeutic agents.

The invention will first be described in terms of a preferred embodiment, the treatment of type I diabetes mellitus.

### Type I Diabetes Mellitus

The pathology of diabetes mellitus can be attributed to three major effects of insulin insufficiency: (1) a decrease in the utilization of glucose by the body cells, causing an increase in blood glucose to supranormal levels; (2) a marked increase in the mobilization of fats from the fat storage areas, causing abnormal fat metabolism as well as the deposition of lipids in vascular walls to cause atherosclerosis and (3) depletion of protein in the tissues of the body. Diabetes is a chronic disease that presently has no cure. Alleviation of the pathological effects of diabetes would be assisted by partially or completely overcoming insulin deficiency.

In a preferred embodiment, the invention provides for the stable, long-term expression of insulin in liver cells using a viral vector delivery system. This allows for the resultant reversal of diabetes in patients treated by the method of the invention, as is demonstrated herein using chemically-induced diabetic rats. Thus, the invention provides a method of delivering an insulin gene directly to a patient's own liver cells which regulates insulin secretion in response to glucose and other substances that stimulate insulin secretion, thereby improving control of blood glucose without the need for immunosuppression.

In this manner one or more of the disadvantages of current treatments for type I diabetes mellitus may be overcome or substantially ameliorated by genetically engineering, from the patient's own cells, an "artificial beta cell" to mimic the function of pancreatic cells, i.e., a non-islet cell capable of synthesising, storing and secreting mature insulin in response to metabolic stimuli, such as glucose. The preferred target cells in this method are liver cells which express glucokinase and the glucose transporter GLUT 2, as do pancreatic beta cells. As shown herein, as these cells are not true pancreatic cells nor are they transdifferentiated pancreatic beta cells, the target cells are not subject to the autoimmune destruction characteristic of type I diabetes mellitus.

### Nucleic acid encoding insulin

The product of the insulin gene, proinsulin, must be cleaved at specific points to allow the production of the active hormone, insulin. Briefly, insulin is normally processed into the mature active "A" and "B" chain complex in secretory vesicles of pancreatic beta cells which contain the required processing enzymes, principally proconvertases PC1 and PC2. During this processing, the "C" peptide, which is between the "A" and "B" peptides in proinsulin is excised by the proconvertases which make two separate cleavages, one at the B-C junction and the other at the C-A junction. Liver cells do not normally possess insulin proconvertases PC1 and PC2 that cleave human proinsulin to active insulin and c-peptide. Thus transfection which results in the expression of proinsulin in the absence of the proconvertases or an alternative mechanism to cleave proinsulin to insulin offers no potential for reduction of blood glucose levels. In the absence of the proconvertases, a number of approaches have been reported in which the recombinant insulin gene is engineered in a manner whereby the expressed proinsulin may be cleaved by the constitutive secretory pathway.

To allow human proinsulin to be processed by the constitutive pathway Groskreutz et al. ("Genetically engineered proinsulin constitutively processed and secreted as mature, active insulin" J. Biol. Chem. 1994, Feb 25;269(8):6241-6245) introduced furin-cleavage sites (Arg-X-(Lys/Arg)-Arg) at both B-C and C-A junctions of human proinsulin. In this manner the proinsulin is capable of being correctly processed by the endogenous Golgi-anchored processing enzyme furin which is present in the constitutive pathway of most cells, including liver cells. Groskreutz *et* al (1994 *ibid*) reported that following transfection the variant human proinsulin cDNA was processed constitutively into active mature human insulin. A similar approach has been reported by Falqui et al ("Reversal of diabetes in mice by implantation of human fibroblasts genetically engineered to release mature human insulin" Human Gene Therapy 1999 Jul 20;10(11):1753-1762; Falqui L, et al., "Human proinsulin production in primary rat hepatocytes after retroviral vector gene transfer" J. Mol. Med. 1999 Jan;77(1):250-253). Thus, the proconvertases PC1 and PC2 are not necessarily required for *in vivo* proinsulin conversion in primary hepatocytes or many other cell types, so long as the proinsulin polypeptide is engineered to be correctly cleaved by a generic proprotein convertase, such as by alteration of the dibasic processing sites on the proinsulin molecule to be recognised by the generic proprotein convertase, furin.

In the context of the present invention as it relates to treatment of type I diabetes mellitus, therefore, the nucleic acid molecule may comprise any nucleic acid sequence which encodes insulin or a polypeptide capable of being processed, for example by post-translational cleavage, to a polypeptide having biological activity of insulin. In this context a desired biological activity of insulin is an ability to regulate blood glucose levels. Examples include furin-cleavable insulin sequences, including preproinsulin and proinsulin sequences, such as sequences described in Groskreutz *et al* (1994 *ibid*), United States Patent No. 6,348,327 dated 19 February, 2002 to Gorman and Groskreutz and entitled "Non-endocrine animal host cells capable of expressing variant proinsulin and processing the same to form active, mature insulin and methods of culturing such cells"; Falqui *et al* (1999 *ibid*), Hosaka et al., "Arg-X-Lys/Arg-Arg motif as a signal for precursor cleavage catalyzed by furin within the constitutive secretory pathway" J. Biol. Chem. 1991 Jul 5;266(19):12127-12130; Gros et al., "Regulated production of mature insulin by non-beta-cells" Hum. Gene Ther. 1997 Dec 10;8(18):2249-2259; Barry et al., "Glucose-regulated insulin expression in diabetic rats" Human Gene Therapy 2001 Jan 20;12(2):131-139; and United States Patent Application No. 20030032144, to Tatake et al*.* entitled "Nucleic acid constructs useful for glucose regulated production of human insulin in somatic cell lines" published February 13, 2003, and sequences described in Simonson et al., "Synthesis and processing of genetically modified human proinsulin by rat myoblast primary cultures" Human Gene Therapy 1996 Jan;7(1):71-78; Alam & Sollinger., "Glucose-regulated insulin production in hepatocytes" Transplantation 2002 Dec 27;74(12):1781-1787; Polastri et al., "Effects of carboxypeptidase E overexpression on insulin mRNA levels, regulated insulin secretion, and proinsulin processing of pituitary GH3 cells transfected with a furin-cleavable human proinsulin cDNA" Cell Transplantation 2002;11(8):803-811; Auricchio et al., "Constitutive and regulated expression of processed insulin following in vivo hepatic gene transfer" Gene Therapy 2002 Jul;9(14):963-971; Shaw et al., "Secretion of bioactive human insulin following plasmid-mediated gene transfer to non-neuroendocrine cell lines, primary cultures and rat skeletal muscle in vivo" J. Endocrinol. 2002 Mar;172(3):653-672; Short et al., "Adenovirus-mediated transfer of a modified human proinsulin gene reverses hyperglycemia in diabetic mice" Amercian J. Physiol. 1998 Nov;275(5 Pt 1):E748-56, each of which is incorporated herein by reference.

Hay and Docherty (2003) examined amino acid substitutions used to create furin consensus sequences and showed that the addition of basic residues at the C-peptide/A-chain junction was responsible for reduced production of furin-cleavable proinsulin relative to that of an unmodified proinsulin construct. Using this information, Hay and Docherty engineered a cDNA for furin-cleavable rat proinsulin I that was efficiently processed to mature insulin and expressed at the same level as wild-type proinsulin (Hay and Docherty, "Enhanced expression of a furin-cleavable proinsulin" J Mol Endocrinol. 2003 Dec;31 (3):597-607). The skilled addressee will appreciate that improved versions of furin-cleavable insulin sequences, such as described by Hay and Docherty (2003) and the application of the principles therein to alternative insulin and proinsulin sequences such as human sequences, are also contemplated within the present invention.

In the context of the present invention as it relates to treatment of type I diabetes mellitus, it will also be understood that functional variants of insulin may be utilised. For example, a change of His-10 to Asp in the B peptide may be made so as to enhance stability of the mature insulin product (Groskreutz *et al.,* 1994 *ibid).* Further examples of functional variants of insulin, and precursors thereof, are included in the documents listed herein, the contents of which are incorporated by reference.

### Vectors

A problem encountered in any gene therapy approach involving liver cells, is that liver cells are not easy to transfect or transduce with genes, as they are quiescent non-dividing cells. Many vector systems, such as Moloney murine retroviral vectors lead to low levels of insulin production (Kolodka *et al* 1995, *ibid),* which are unlikely to be feasible in therapeutic situations. Further, as retrovirus-based vectors can only transduce actively dividing cells, it is necessary to stimulate division in the normally quiescent liver cells by partial hepatectomy before retrovirus infusion (Muzzin et al "Hepatic Insulin Gene Expression as Treatment for Type 1 Diabetes Mellitus in Rats" Molecular Endocrinology 11 (6): 833-837 1997).

Previous studies have used adenoviral vectors because of their ability to deliver genes to non-dividing cells, such as primary liver cells. Adenoviral vectors, however, are not the appropriate gene transfer method for liver cells for several reasons: the genes remain episomal and are not incorporated, expression is transient (typically from 10 days up to six weeks only) and a strong host immune response is elicited. Of particular concern is the development of cytotoxic T-lymphocytes directed against viral proteins that lyse cells expressing the recombinant genes, thus limiting the persistence of gene expression. There are also safety concerns when large volumes of adenoviral vectors are used in a clinical situation.

Lentiviral vectors have been reported to deliver genes to primary liver cells efficiently and permanently, integrating into the genome of non-dividing cells such as primary liver cells (Lewis and Emerman "Passage through mitosis is required for oncoretroviruses but not the immunodeficiency virus" J. Virol. 1994, 68:510-6). Lentiviral vectors also do not suffer from the same transcription silencing mechanism, as do Moloney Murine Leukaemia virus (MLV) retroviral vectors. Lentiviruses differ from other retroviruses in that they have karyophilic determinants contained in several virion proteins, such as matrix or VPR, which interact with the nuclear import machinery and mediate active transportation of the viral pre-integration complex through the nucleopore. Therefore, lentiviral integration into the genome of the host cells is not dependent on cell division.

In a preferred method of the present invention the viral vector system is a lentiviral vector.

Early generation lentiviral vectors have been shown to deliver genes to non-dividing cells in a number of systems. As demonstrated in the Examples herein one such early generation lentiviral vector, the pHR'CMV vector (Blomer et al., "Highly efficient and sustained gene transfer in adult neurons with a lentivirus vector" J. Virol. 1997, 71:6641-49), when used in the method of the invention, is capable of inducing gene expression in liver cells.

Choi et al ("Hybrid HIV/MSCV LTR enhances transgene expression of lentiviral vectors in human CD34(+) hematopoietic cells" Stem Cells 2001;19(3):236-46) have described a new lentiviral vector in which the CMV enhancer/promoter was deleted and a portion of the U3 region of the 3' HIV long terminal repeat (LTR) was replaced with the U3 region of murine stem cells virus (MSCV) LTR. This has also been described in United States of America Patent No. 6,218,186 dated 17 April 2001 to Choi and Gewirtz and entitled "HIV-MSCV hybrid viral vector for gene transfer". Each of these documents is incorporated herein by reference. This vector has been shown to enhance gene expression in haematopoietic stem cells *in vitro* but has not previously been demonstrated to transduce liver cells.

This vector has a high level of expression and has not been used previously in other studies for this purpose.

As demonstrated in the Examples herein, lentiviral vectors when used in the method of the invention are capable of inducing high levels of gene expression in liver cells. Preferred lentiviral vectors are described in Choi *et al* (2001, *ibid)* or in United States of America Patent No. 6,218,186.

### Surgical procedure

Previous attempts at surgical transfusion of vectors have been reported, however each is associated with one or more deficiencies. Tomori, H., et al ('In situ perfusion of the liver enhances the efficiency of retrovirus - mediated gene transfer to hepatocytes', Eur. Surg. Res., 2000: 32: 18-22) and Futagawa Y, et al. ("Efficiency of adenovirus-mediated gene transfer into hepatocytes by liver asanguineous perfusion method" Res Exp Med. 199, 263-274, 2000) propose a method that involves making an incision in the wall of the infrahepatic vena cava (IVC) and the subsequent insertion of a cannula. However, this can easily cause IVC narrowing and pelvic limb thrombosis.

Isabelle Lorand, et al ('Portal branch ligation induces efficient retrovirus - mediated gene delivery in rat liver', J. Gene Med., 2004: 6: 507-513.) used a method of ligating part of the portal branch instead of 70% hepatectomy to induce transduction in rat liver, which would be expected to cause problems with liver function and other syndromes. In addition, this is similar to performing a partial hepatectomy and is unacceptable for clinical application.

In the surgical procedure of the present invention, blood flow to the liver is inhibited during infusion of the vector into the liver. Blood flow to the liver may be inhibited by any suitable means which restricts, or preferably stops, blood flow to and from the liver. In a preferred form, blood flow to the liver may be inhibited by ligating or clamping the right adrenal vein of the liver, and successively clamping the infrahepatic vena cava, the hepatic artery, the portal vein, and the suprahepatic vena cava.

The skilled addressee will appreciate that non-inventive modifications may be made to the procedure. It is noted, for example, that visualisation of the right adrenal vein during surgical procedure may be difficult when performed on a small animal, such as a mouse or rat. Accordingly, the preferred surgical method for small animals involves ligation of the right adrenal vein. In this manner, ligation of the right adrenal vein of the small animal is preferably permanent. As described herein the permanent ligation of the right adrenal vein has not been observed to have any apparent deleterious effects on the experimental animals as they have remained healthy for over 240 days post-operative, with reversal of symptoms of diabetes. In larger animals such as humans the larger size of the right adrenal vein would be expected to be conducive to easier visualisation during the surgical procedure. Accordingly, the preferred surgical method for larger animals, such as humans, involves clamping of the right adrenal vein. In this manner the right adrenal vein may be released at an appropriate time during, at, or after, completion of, the surgical procedure. Preferably, the right adrenal vein is released after restoration of the blood flow to the suprahepatic vena cava. More preferably, the right adrenal vein is released after restoration of the blood flow to the suprahepatic vena cava and before restoration of blood flow to the infrahepatic vena cava.

The period of time over which blood flow to the liver is inhibited may depend on the particular circumstances of the procedure being undertaken and may be determined by the skilled addressee using the description herein as a guide, taking into account factors such as the particular recipient mammalian species, the size of the recipient mammal and recipient liver, the general state of health of the recipient mammal, the time required to permit adequate infusion of the vector and minimisation of risk of damage to the liver by excessive inhibition of blood flow. For example, it would be expected that the time required for infusion of the vector into the liver of a large mammal, such as a human, might be greater than the time required for infusion of the vector into the liver of a smaller mammal. As a guide, on the basis of the Examples herein, the following periods of time are appropriate for a small mammal, such as a mouse or rat.

Inhibition of blood flow may be for any suitable period of time, such as any period of time between about one minute and about ten minutes. For example, inhibition of the blood flow may be for about 1 minute, about 2 minutes, about 3 minutes, about 4 minutes, about 5 minutes, about 6 minutes, about 7 minutes, about 8 minutes, about 9 minutes, or about 10 minutes.

For the infusion the lentiviral vector may be in any suitable physiologically acceptable form, such as in a physiologically acceptable solution such as a saline solution, a buffer or a cell culture medium. Optionally, additional agents such as excipients, carriers, and/or adjuvants may be present.

The vector is infused, or perfused, into the liver by any suitable means, such as through a suitable vessel, such as via a cannula inserted into the portal vein. Alternatively, infusion through pumping, syringe, drip or gravity feed may be appropriate. The skilled addressee will be able to determine suitable rate(s) of infusion using the description herein as a guide. As a guide, on the basis of the Examples herein, an infusion rate of approximately 15-20 seconds per ml delivered by syringe is appropriate for a small mammal, such as a mouse or rat.

For infusion the lentiviral vector may be used in any suitable amount which will provide the desired outcome, such as partial or complete correction of diabetes. A suitable amount of lentiviral vector administered to provide a desired outcome may depend on the particular circumstances of the procedure being undertaken and may be determined by the skilled addressee using the description herein as a guide, taking into account factors such as the particular recipient mammalian species, the size of the recipient mammal and recipient liver, the general state of health of the recipient mammal, and minimisation of risk of damage to the liver by the procedure.

As a guide, administration of the lentiviral vector for a small mammal such as a mouse or rat, may be in an amount between at least about 0.5x10⁵ and at least about 8x10⁶ transduction units. For example, the amount administered may be about 0.5x10⁵, about 1x10⁵, about 2x10⁵, about 3x10⁵, about 4x10⁵, about 5x10⁵, about 6x10⁵, about 7x10⁵, about 8x10⁵, about 9x10⁵, about 1x10⁶, about 2x10⁶, about 3x10⁶, about 4x10⁶, about 5x10⁶, about 6x10⁶, about 7x10⁶, or about 8x10⁶ transduction units. As a further guide, the total amount administered to a mouse may be at least about 1x10⁵ to 2.5x10⁵ transduction units and the total amount administered to a rat may be about 4x10⁶ transduction units.

The vector is administered in any suitable volume which will provide the desired outcome, such as partial or complete correction of diabetes. A suitable volume may depend on the particular circumstances of the procedure being undertaken and may be determined by the skilled addressee using the description herein as a guide. As a guide, for a small mammal such as a rat or mouse, a volume in the range of about 0.2ml to about 5ml, such as about 0.2ml, about 0.3ml, about 0.4ml), about 0.5ml, about 0.6ml, about 0.7ml, about 0.8ml, about 0.9ml, about 1ml, about 2ml, about 3ml or about 4ml may be used.

One or more infusions of vector may be performed. If one infusion only is performed, blood flow to the liver is permanently restored after completion of the infusion. If more than one infusion is desired, such as two, three, four or five infusions, blood flow to the liver is temporarily restored after each infusion.

As a further guide, for a mouse about 1x10⁵ to 2.5x10⁵ transduction units may be administered, for example in a volume of about 0.5ml to about 0.7ml, optionally delivered in one, two or three approximately equal infusions. As a further guide, for a rat about 4x10⁶ transduction units may be administered, for example in a volume of about 4ml, optionally delivered in one, two or three approximately equal infusions.

Temporary restoration of blood flow to the liver may be for a suitable period of time to allow liver cells to be bathed in blood. This permits the infused vector to be distributed through the liver by the blood flow, thereby increasing the exposure of liver cells to the vector. The period of time over which blood flow to the liver is temporarily restored may depend on the particular circumstances of the procedure being undertaken and may be determined by the skilled addressee using the description herein as a guide, taking into account factors such as the particular recipient mammalian species, the size of the recipient mammal and recipient liver, the general state of health of the recipient mammal, the time required to permit liver cells to be bathed in blood and minimisation of risk of damage to the liver by the procedure. For example, it may be expected that the time allowed for temporary restoration of the blood flow to the liver of a larger mammal, such as a human, might be approximately the same or greater than the time allowed for a smaller mammal. As a guide, on the basis of the Examples herein, the following periods of time are appropriate for a small mammal, such as a mouse or rat.

Temporary restoration of blood flow to the liver may be for any period of time between about 1 minute and about 5 minutes. For example, the period of time may be about 1 minutes, or about 2 minutes, or about 3 minutes, or about 4 minutes, or about 5 minutes. In one embodiment, the suitable time is any period of time between about 2 minutes and about 3 minutes.

Restoration of blood flow to the liver, be it permanent or temporary restoration, may be by any suitable means, such as by successively releasing the clamped vessels in any order that allows blood to infuse into the liver. During restoration of blood flow to the liver the vector will be distributed through the liver. During restoration of blood flow it is desirable to allow the blood to infuse slowly into the liver as this may minimise stress on the liver tissue. As an example, restoring blood flow to the liver may comprise successively restoring blood flow to the suprahepatic vena cava, the infrahepatic vena cava, the portal vein and the hepatic artery.

Where more than one infusion of vector is desired, after temporary restoration of blood flow to the liver, blood flow to the liver is again inhibited by any suitable means, such as described above. Vector infusion is repeated, following which blood flow to the liver is again restored, temporarily or permanently depending on whether the infusion is again to be repeated additional times. The infusions may be repeated any suitable number of times to achieve the desired efficiency of transduction. It will be remembered that it is also desirable to minimise the amount of unnecessary stress on the animal or patient and this may influence the number of infusions. As a guide, the infusions may be repeated one, two, three or four times, thus providing a procedure in which one, two, three, four or five infusions are performed.

As described more specifically in the Examples, the inventors demonstrate the method for the *in vivo* delivery of lentiviral vectors containing one or more gene(s) of interest, for example enhanced green fluorescent protein (EGFP) or furin-cleavable insulin, to recipient mammals, such as adult Wistar rats, in the form of a procedure of portal infusion, whereby the liver is segregated from normal blood supply by ligating the right adrenal vein and successively clamping the infrahepatic vena cava, hepatic artery, portal vein and suprahepatic vena cava in turn and infusing the vector through a cannula inserted in the portal vein.

During this procedure there is no blood supply to the liver, for example for a period of five minutes. The clamps are then removed, for example for a period of two minutes, to allow blood supply to the liver. This procedure may be repeated, for example for one, two, three or four times for maximal transduction. As demonstrated in the Examples herein, this procedure segregates gene expression to the liver alone. An embodiment of the surgical procedure is illustrated in **Figure 1****.**

This surgical procedure is unique and, as demonstrated in the Examples herein, results in long term high levels of expression of the transduced gene.

### Other Conditions

Whilst the invention is herein explicitly exemplified in terms of type I diabetes, the skilled addressee will readily appreciate that the method of the invention, in respect of the infusion of liver cells of a patient with an appropriate vector comprising an appropriate gene, will be applicable to the treatment of such conditions by replacement of the furin-cleavable insulin gene, as exemplified herein for the treatment of type I diabetes mellitus, with an appropriate nucleic acid molecule, such as a nucleic acid sequence encoding an appropriate gene or genes, optionally together with a suitable promoter and/or enhancer sequences as appropriate. Selection of the nucleic acid sequence, such as a nucleic acid sequence encoding a desired gene product, is dependent on the specific condition to be treated. Such selection lies within the skill of the art.

Conditions that may be susceptible to treatment by the methods of the invention include conditions in which the expression of a gene is increased or is decreased, conditions where the activity of a protein is increased or decreased or otherwise altered in the condition to be treated. Therefore, a nucleic acid molecule utilised in the method of the invention may alleviate or rectify a condition by, as appropriate, decreasing or increasing expression of a gene associated with the condition, decreasing or increasing the activity of a gene product associated with the condition or otherwise countering the alteration associated with the condition.

A number of non-limiting examples of conditions that may be treated by the methods of the invention will now be described.

### Familial Hypercholesterolemia

Examples of diseases or conditions which may be treated by the methods and materials of the invention include treatment of individuals affected by familial hypercholesterolemia, an autosomal dominant disorder due to a mutation in the low-density lipoprotein receptor (LDLR) gene. Although lowering plasma cholesterol decreases the risk of coronary artery disease, patients with familial hypercholesterolemia generally respond poorly to pharmacologic treatment. A number of publications have proposed gene therapy approaches to the treatment of familial hypercholesterolemia (for example, Shichiri, M et al "Intravenous gene therapy for familial hypercholesterolemia using ligand-facilitated transfer of a liposome:LDL receptor gene complex", Gene Ther. 2003 May;10(9):827-31). Grossman et al. ("A pilot study of ex vivo gene therapy for homozygous familial hypercholesterolaemia" Nat Med. 1995 Nov;1(11):1148-1154) report a study in which 5 patients were transplanted with retrovirally transduced hepatocytes. When adenoviral vectors have been used in animal systems the transience of the effectiveness of the procedure has been linked to strong adverse immune reactions against vector antigens, the loss of therapeutic DNA in physiological cell turnover, as the genes did not incorporate (Yang et el., "MHC class I-restricted cytotoxic T lymphocytes to viral antigens destroy hepatocytes in mice infected with E1-deleted recombinant adenoviruses" Immunity 1994 Aug;1(5):433-42; Yang et al., "Cellular and humoral immune responses to viral antigens create barriers to lung-directed gene therapy with recombinant adenoviruses" J. Virol. 1995 Apr;69(4):2004-15).

All such approaches, however, lack an efficient means of delivering the correct gene to the liver cells. Such patients therefore would clearly benefit from improved methods of alleviating the condition, such as gene therapy approaches which provide for long term alleviation.

In one embodiment, the present invention provides a means of therapy of familial hypercholesterolemia. In this embodiment the lentiviral vector, such as a lentiviral vector described herein, may comprise a nucleic acid sequence encoding a low density lipoprotein receptor. For example, the vector may comprise a nucleic acid sequence encoding an LDLR, such as described in Shichiri *et al* (2003 *ibid),* Grossman *et al* (1995 *ibid*), Yang *et al* (1994 *ibid;* 1995 *ibid;* Yang et al.," Thrombospondin-1 mediates distal tubule hypertrophy induced by glycated albumin" Biochem J. 2004 Apr 1;379(Pt 1):89-97).

### Hemophilia

A further example of a condition susceptible to treatment by the materials and methods of the present invention is hemophilia. There are two types of hemophilia, A and B (Christmas Disease). Low levels or complete absence of a blood protein essential for clotting causes both. Patients with hemophilia A lack a protein known as factor VIII whilst those with hemophilia B lack factor IX. Current treatments for hemophilia include periodic transfusion with blood products or recombinant versions of the required blood factor. However, despite improved screening of donor blood the risk of viral infection through blood or blood product transfusion remains a deficiency of this treatment. Furthermore, with both blood product-derived and recombinant-derived factor VIII or IX therapy, the development of antibodies that block the activity of the clotting factors has complicated treatment for some patients. Hemophilia has been considered an ideal candidate for gene therapy as a modest rise of only a few percent of normal levels of Factor VIII or Factor IX has been suggested to be sufficient to ameliorate many of the clinical consequences of haemophilia A or B, respectively. Consequently a number of publications have proposed gene therapy approaches to treatment of hemophilia.

To date, attempts at gene therapy for hemophilia have predominantly been performed with adeno-associated vectors- and suffer from a general problem in that while most hepatocytes take up the vector only about 5% expression is observed, there have also been other problems with immune tolerance.

The results presented herein demonstrate the efficacy of the present invention in the treatment of type I diabetes, through a surgical method for the introduction of a lentiviral vector comprising a sequence encoding a polypeptide of benefit in the treatment of the condition. By adjusting the composition of the lentiviral vector, the method of the invention may find efficacy in the treatment of hemophilia.

Thus, in one embodiment the present invention provides a method for the treatment of hemophilia. In this embodiment the lentiviral vector may comprise a nucleic acid sequence encoding Factor VIII or Factor IX. Such sequences are known in the art and include sequences described in, for example, Burton et al., "Coexpression of factor VIII heavy and light chain adeno-associated viral vectors produces biologically active protein" Proc. Natl. Acad. Sci. U S A. 1999 Oct 26;96(22):12725-30; Chao et al., "Sustained expression of human factor VIII in mice using a parvovirus-based vector" Blood 2000 Mar 1;95(5):1594-9; Chao et al., "Expression of human factor VIII by splicing between dimerized AAV vectors" Mol. Ther. 2002 Jun;5(6):716-22; Chao and Walsh., "Induction of tolerance to human factor VIII in mice" Blood 2001 May 15;97(10):3311-2; Conto L et al Blood 2002 100, Abstract 431; Wang et al., "Sustained correction of bleeding disorder in hemophilia B mice by gene therapy" Proc. Natl. Acad. Sci. U S A. 1999 Mar 30;96(7):3906-10; Wang et al., "Sustained expression of therapeutic level of factor IX in hemophilia B dogs by AAV-mediated gene therapy in liver" Mol. Ther. 2000 Feb;1(2):154-8; Mount et al., "Sustained phenotypic correction of hemophilia B dogs with a factor IX null mutation by liver-directed gene therapy" Blood 2002 Apr 15;99(8):2670-6; Kay et al Blood 2002 100 Abstract 426; Kay et al., "B-CLL: is the enigma of disease heterogeneity about to be revealed?" Blood 2002 Aug 15;100(4):1110-1. Optionally, the lentiviral vector may further comprise one or more promoters and/or enhancer sequences, such as hepatocyte-specific promoters, as described for example in Follenzi et al., "Efficient gene delivery and targeted expression to hepatocytes in vivo by improved lentiviral vectors" Human Gene Therapy 2002 Jan 20;13(2):243-60.

### Alpha-1-antitrypsin (AAT) deficiency

A further example of a condition susceptible to treatment by the materials and methods of the present invention is the common monogenic lung disease Alpha-1-antitrypsin (AAT) deficiency.

AAT is produced in the liver and circulated to the lung where it protects elastin fibers and other connective tissue components of the alveolar wall from degradation by neutrophil elastase. AAT deficiency may lead to death due to obstructive pulmonary disease. The gene encoding AAT has been isolated and infusion with recombinant human AAT protein represents one form of current therapy. A number of studies have shown promise toward the development of therapeutic approaches based on gene therapy. In general these approaches have utilised adeno-associated virus vectors and demonstrate, for example, transduction of skeletal muscle cells to express AAT (for example, see Song et al, "Sustained secretion of human alpha-1-antitrypsin from murine muscle transduced with adeno-associated virus vectors", Proc. Natl. Acad. Sci. USA Vol. 95, 14384-14388, 1998). By incorporation of a nucleic acid sequence encoding AAT, or a functional variant thereof, into a lentiviral vector, such as a lentiviral vector described herein, and transduction of liver cells through the methods of the present invention long term stable expression of AAT may be achieved.

Thus, in a further embodiment the present invention provides a method for the treatment of alpha-1-antitrypsin (AAT) deficiency. In this embodiment the lentiviral vector may comprise a nucleic acid sequence encoding AAT or a functional variant thereof. As noted above, selection of an appropriate nucleic acid sequence encoding AAT or a functional variant thereof is within the skill in the art. For example, Song *et al* (1998 *ibid*) describes a 1.8kb human AAT cDNA sequence. Other sequences encoding functional AAT are known in the art and include, for example, sequences described in Song et al., "Effect of DNA-dependent protein kinase on the molecular fate of the rAAV2 genome in skeletal muscle" Proc. Natl. Acad. Sci. U S A. 2001 Mar 27;98(7):4084-8; Song et al., "Stable therapeutic serum levels of human alpha-1 antitrypsin (AAT) after portal vein injection of recombinant adeno-associated virus (rAAV) vectors" Gene Ther. 2001 Sep;8(17):1299-1306; Song et al., "Ex vivo transduced liver progenitor cells as a platform for gene therapy in mice" Hepatology 2004 Oct;40(4):918-24; Zhang et al., "Alpha-1-antitrypsin expression in the lung is increased by airway delivery of gene-transfected macrophages" Gene Therapy 2003 Dec;10(26):2148-52 and Ferkol et al., "Transfer of the human Alphal-antitrypsin gene into pulmonary macrophages in vivo" Am. J. Respir. Cell Mol. Biol. 1998 May;18(5):591-601.

### Crigler Najar syndrome Type I or Type II

A further example of a condition susceptible to treatment by the materials and methods of the present invention is Crigler Najar syndrome Type I or Type II (a lower than normal (type II) or absent activity of hepatic bilirubin UDP-glucuronyltransferase (type I). Hence, in a further embodiment of the present invention there is provided a method for the treatment of Crigler Najar syndrome Type I or Type II. In this embodiment the lentiviral vector may comprise a nucleic acid sequence encoding hepatic bilirubin UDP-glucuronyltransferase, or a functional variant thereof, such as UGT1A1 as described for example in Gong QH, et al "UDP glucuronosyltransferase genes are encoded at the human UGT1 gene complex locus", Pharmacogenetics 2001: 11, 357-68.

From the foregoing discussion the skilled addressee will appreciate that indeed any of the list of liver diseases that result from single nucleotide mutations are also logical targets and even anti tumour responses may be targeted with the appropriate tumour-specific marker.

### Genetic prodrug activation therapy

The method of the invention also finds utility in genetic prodrug activation therapy (or suicide gene therapy) for the treatment of hepatic cancer as well as a variety of other diseases of the liver. Genetic prodrug activation therapy involves the insertion of a gene encoding a drug-metabolizing enzyme into cells and the systemic administration of a prodrug, such as by systemic administration. The prodrug is converted to a cytotoxic agent by the action of the expressed enzyme. For example, the herpes simplex virus thymidine kinase gene product converts gangcyclovir into a toxic metabolite that kills transduced cells. To ensure that the drug-metabolizing enzyme is only expressed in the targeted subset of cells, the transcriptional apparatus of a gene that is unique to this subset may be used to regulate the gene encoding the drug-metabolizing enzyme. A major obstacle to successful clinical application of this type of therapy has been inefficient delivery of genes to the tumour cells. As demonstrated herein, the method of the present invention provides an efficient delivery system for the targeted delivery of specific nucleic acid sequences to liver cells and the stable expression of the encoded gene product, such as a drug-metabolising enzyme for genetic prodrug activation therapy.

In this aspect of the invention the desired selectivity in gene expression can be achieved by a variety of means. For example, increasing the specificity of gene delivery such as by addition of tumour-specific ligands to gene therapy vectors or monoclonal antibodies recognising tumour antigens such as AF-20 may result in an improved selectivity of gene transfer (Mohr et al 1999 Hepatology 29, 82-89). As a further example, by controlling gene expression with tumour-specific promoters such as α-fetoprotein or the use of a radioinducible promoter activated by the selective irradiation of the tumour tissue: for example, α-fetoprotein- since many primary express α-fetoprotein, assigning the transcriptional control of the therapeutic gene to the α-fetoprotein promoter results in gene expression specifically within liver tumours (Kaneko et al., "Adenovirus-mediated gene therapy of hepatocellular carcinoma using cancer-specific gene expression" Cancer Res. 1995 Nov 15;55(22):5283-7; Su et al., "Selective killing of AFP-positive hepatocellular carcinoma cells by adeno-associated virus transfer of the herpes simplex virus thymidine kinase gene" Human Gene Therapy 1996 Mar 1;7(4):463-70); or for example, a radioinducible promoter (Kawashita et al., "Regression of hepatocellular carcinoma in vitro and in vivo by radiosensitizing suicide gene therapy under the inducible and spatial control of radiation" Human Gene Therapy 1999 Jun 10;10(9):1509-19. The two main approaches to gene therapy of liver cancer aim at killing directly malignant cells or improving the host's defensive systems

### Nucleic Acid Molecules

As will be appreciated from the description herein, the nucleic acid molecule may be any desired nucleic acid molecule. For example the nucleic acid molecule may comprise a gene encoding an expression product of interest, such as a polypeptide. The gene may encode a secreted or non-secreted protein, or an active portion thereof. The selection of a suitable gene will be apparent to those skilled in the art. By "gene" is meant DNA that encodes a desired product, such as, for example, a cytokine, a clotting factor, a hormone, an enzyme, a transport protein, a regulatory protein, a structural protein, a receptor, an antigen, etc. Representative examples of genes for introducing into human liver cells are those encoding human erythropoietin (for example as described in United States of America Patent No. 4,703,008 dated 27 October 1987 to Lin and entitled "DNA sequences encoding erythropoietin"), human G-CSF, human GM-CSF (for example as described in Cantrell et al., "Cloning, sequence, and expression of a human granulocyte/macrophage colony-stimulating factor" Proc Natl Acad Sci U S A. 1985 Sep;82(18):6250-4), plasminogen activator, urokinase, insulin (for example human insulin as described in United States of America Patent No. 4,652,525 dated 24 March 1987 to Rutter et al and entitled "Recombinant bacterial plasmids containing the coding sequences of insulin genes" or proinsulin described in United States of America Patent No. 4,431,740 dated 14 February 1984 to Bell et al and entitled "DNA Transfer vector and transformed microorganism containing human proinsulin and pre-proinsulin genes" or furin-cleavable insulin as described in Groskreutz *et al,* 1994 *ibid*), interleukins (for example interleukin-1, interleukin-2 [for example as described in United States of America Patent No. 4,738,927 dated 19 April 1988 to Taniguchi et al and entitled "Gene coded for interleukin-2 polypeptide, recombinant DNA carrying the said gene, a living cell line possessing the recombinant DNA, and method for producing interleukin-2 using the said cell"], interleukin-3 [for example as described in EP Publ. 275,598 and 282,185], interleukin-4, interleukin-7 [for example as described in United States of America Patent No. 4,965,195 dated 23 October 1990 to Namen et al entitled "Interleukin-7"], etc.), interferons, Factor VIII, Factor IX, von Willebrand Factor, ADA, human growth hormone (for example as described in United States of America Patent No. 4,342,832 dated 3 August 1982 to Goeddel et al and entitled "Method of constructing a replicable cloning vehicle having quasi-synthetic genes"), etc., analogs and fusions thereof (for example fusions of GM-CSF and IL-3 [for example as described in United States of America Patent No. 5,108,910 dated 28 April 1992 to Curtis et al and entitled "DNA sequences encoding fusion proteins comprising GM-CSF and IL-3"]. Each of the foregoing patents and publications is expressly incorporated herein by reference.

As will be apparent from the foregoing, the polypeptide may be any polypeptide the expression of which in the liver cells of the recipient mammal may benefit the health of the mammal, such as a polypeptide capable of alleviating a deficiency in the expression of an endogenous gene. A polypeptide capable of alleviating a deficiency in the expression of an endogenous gene may be a polypeptide capable of alleviating a quantitative deficiency of said endogenous gene product, such as a deficiency of insulin, or a qualitative deficiency of said endogenous gene product, such as alleviating conditions associated with expression of an abnormal endogenous gene sequence such as factor VIII, factor IX, low-density lipoprotein receptor (LDLR) gene, alpha-1-antitrypsin (AAT). Any other liver diseases resulting from a mutation that the correction of would cure or alleviate the disease.

The nucleic acid molecule may encode a gene product which provides a functional gene or gene product for the treatment of a mammal having a condition associated with expression of an abnormal gene or abnormal expression of a gene. As described herein the delivery method is appropriate for the delivery of any gene(s) of interest to the liver of a recipient mammal. For example, the gene of interest may be a gene encoding a functional protein or polypeptide the expression of which in a liver cell of the mammal complements a protein or polypeptide deficiency which is associated with a disease state or condition in the recipient mammal. Examples of genes of interest include, but are not limited to, genes encoding functional gene products of monogenic liver disorders, such as genes encoding insulin, low density lipoprotein receptor, alpha 1 antitrypsin, factor VIII, factor IX. It will be understood that in the context of the invention reference to genes or nucleic acid sequences encoding a particular protein or polypeptide, such as insulin, low density lipoprotein receptor, alpha 1 antitrypsin, factor VIII, factor IX, includes any nucleic acid sequence the expression of which may result in production of the product of interest. As an example, genes encoding insulin include nucleic acid sequences which encode a furin-cleavable insulin since the expression of the furin-cleavable insulin sequence may result in expression of insulin.

The nucleic acid molecule may encode a gene product, such as a polypeptide, for example an enzyme, suitable for genetic prodrug activation therapy. A polypeptide suitable for genetic prodrug activation therapy may be any polypeptide capable of converting a prodrug into a toxic metabolite, such as a herpes simplex virus thymidine kinase which is capable of converting gangcyclovir into a toxic metabolite that kills transduced cells. Other non-limiting examples are described in the following table.

| **Enzyme** | **Prodrug** | **Drug** |
|---|---|---|
| **Non mammalian enzymes** | | |
| Thymidine kinase | gangcyclovir | Gangcyclovir-3P |
| Cytosine deaminase | 5-fluorcytosine | 5-fluorouracil |
| Purine nucleoside phosphorylase | Fluradine MePdR | Active metabolites |
| Carboxypeptidase G2 | CJS278 | Doxorubicin |
| Nitroreductase | CB1954 | Active metabolite |
| Methionine α,γ -lyase | selenomethionine | methylselenol |
| Carboxylesterase | irinotecan | SN-38 |

| **Human enzymes** | | |
|---|---|---|
| Cytochrome P450 | Ifosfamide, cyclophosphamide, ipomeanol | Active metabolites |
| Deoxycytidine kinase | Cytosine arabinoside, gemcitabine | Active metabolites |

Expression of the gene product, such as a polypeptide, in liver cells of the recipient mammal may be associated with no change in the health or behaviour of the recipient mammal or may be associated with an increased or decreased state of health of the recipient mammal compared to the mammal in the absence of expression of the transgene.

The polypeptide may be a polypeptide the expression of which in liver cells of the mammal is associated with a phenotype desirable for experimental investigation. For example, the polypeptide may comprise a marker sequence such as EGFP or LacZ. For example, the polypeptide may be a polypeptide the expression of which in liver cells of the mammal is associated with a decreased or an increased state of health of the recipient mammal. Thus the invention provides methods for the preparation of a mammal capable of expressing a transgene in a liver cell. In some embodiments the recipient mammal may find utility as an experimental mammal, such as an experimental mammalian model of disease. The experimental mammalian model may find utility, for example, in investigation of the disease, such as investigation of the underlying cause of the disease, of the pathology of the disease, of the progression of the disease, and of treatment of the disease, including treatment of symptoms of the disease.

In some embodiments the method may find utility in the treatment of mammalian disease(s), such as a human disease, for example type I diabetes.

The nucleic acid molecule may comprise a nucleotide sequence encoding a nucleic acid-based inhibitor of a gene or of a gene product, such as an inhibitor of transcription or translation of a gene. The nucleic acid-based inhibitor of a gene or of a gene product may be a short interfering RNA (siRNA) sequence or an antisense sequence. siRNA-mediated gene silencing methods, where expression products of a gene are targeted by specific double stranded derived siRNA nucleotide sequences that are complementary to at least a 19-25 nt long segment of the target gene transcript, including the 5' untranslated (UT) region, the ORF, or the 3' UT region, are known in the art (see, for example, PCT publications PCT International Patent Publication No. WO00/44895 (Kreutzer et al entitled "Method and medicament for inhibiting the expression of a defined gene"), PCT International Patent Publication No. WO99/32619 (Fire et al entitled "Genetic inhibition by double-stranded RNA"), PCT International Patent/Publication No. WO01/75164 (Tuschl et al entitled "RNA sequence-specific mediators of RNA interference"), PCT International Patent/Publication No. WO01/92513 (Arndt et al entitled "Methods for mediating gene suppression by using factors that enhance RNAi"), PCT International Patent/Publication No. WO 01/29058 (Mellow and Fire entitled "RNA interference pathway genes as tools for targeted genetic interference"), each incorporated by reference herein in their entirety). Sequences targeted by the siRNA include genes expressing a polypeptide of interest, or an upstream or downstream modulator of such a gene. Examples of upstream or downstream modulators of a gene include, a transcription factor that binds a gene promoter, a kinase or phosphatase that interacts with a polypeptide of interest, and polypeptides involved in regulatory pathways capable of influencing the polypeptide of interest.

As is noted above, the nucleic acid molecule may comprise a nucleotide sequence encoding a nucleic acid-based inhibitor of a gene or of a gene product such as an antisense RNA molecule that, by hybridization interactions, can be used to block expression of a cellular or pathogen mRNA. Alternatively, the RNA molecule can be a ribozyme (e.g., a hammerhead or a hairpin-based ribozyme) designed either to repair a defective cellular RNA or to destroy an undesired cellular or pathogen-encoded RNA (see, e.g., Sullenger., "Revising messages travelling along the cellular information superhighway" Chem. Biol. 1995 May;2(5):249-253; Czubayko et al., "Adenovirus-mediated transduction of ribozymes abrogates HER-2/neu and pleiotrophin expression and inhibits tumor cell proliferation" Gene Therapy 1997 Sep;4(9):943-9; Rossi., "Therapeutic applications of catalytic antisense RNAs (ribozymes)" Ciba Found Symp. 1997;209:195-204; James and Gibson., "The therapeutic potential of ribozymes" Blood 1998 Jan 15;91(2):371-82; Sullenger., "Ribozyme-mediated repair of RNAs encoding mutant tumor suppressors" Cytokines Mol. Ther. 1996 Sep;2(3):201-5; Hampel., "The hairpin ribozyme: discovery, two-dimensional model, and development for gene therapy" Prog. Nucleic Acid Res. Mol. Biol. 1998;58:1-39; Curcio et al., "Oligonucleotides as modulators of cancer gene expression" Pharmacol Therapy 1997;74(3):317-32).

Genes can be inserted into lentiviral vectors used in the methods of the invention using standard methods (see, e.g., Ausubel et al., Current Protocols in Molecular Biology, John Wiley & Sons, New York, N.Y., 1998; Sambrook et al.; Molecular Cloning: A Laboratory Manual; Cold Spring Harbor, N.Y.; (1989). The genes may be inserted so that they are under the control of any appropriate regulatory sequence or sequences, such as lentiviral vector regulatory sequences. Alternatively, the genes can be inserted as part of an expression cassette that includes regulatory elements, such as promoters or enhancers. Appropriate regulatory elements can be selected by one of ordinary skill in this art based on, for example, the desired level of expression. Appropriate regulatory elements may also be selected to include liver-specific promoters to assist limitation of expression of the gene product to liver cells.

As described herein the delivery method is appropriate for the delivery of a gene(s) of interest to the liver of any recipient mammal. The recipient mammal may be any mammalian species of social, economic or research importance including but not limited to members of the genus ovine, bovine, equine, porcine, feline, canine, caprine, as well as primates and rodents. In a preferred embodiment the recipient mammal is a human.

The invention will now be described in greater detail by reference to specific examples, which should not be construed as in any way limiting the scope of the invention.

### Examples

### Example 1: Delivery of insulin-expressing vectors to STZ-induced diabetic Wistar rats and transduction of hepatocytes in vitro

### Materials and Methods

### Construction of HMD/INS-FUR lentiviral vector

Furin-cleavable human proinsulin cDNA (proins.I fur II fur. B10D) (INS-FUR) (Genentech, CA, USA) was cloned into the multi cloning site of the lentiviral hybrid vector HIV/ MSCV (HMD) (Choi et al, 2001 Stem Cells 19: 236-46) at the Eco RI site to produce the HMD/ INS-FUR construct **(****Figure 3****).**

### Generation of Virus

Retroviruses were generated using previously described methods (Naldini, L. et al, 'In vivo gene delivery and stable transduction of nondividing cells by lentiviral vector', Science, 1996: 272: 263-67). The lentiviral vectors were produced by calcium phosphate precipitation cotransfection of three plasmids (10 µg HMD/INS-FUR or 10 µg pHR'CMVGFP (Miyoshi, H., Takahashi, M., Gage, F. H. & Verma, I. M. Stable and efficient gene transfer into the retina using an HIV-based lentiviral vector. Proc. Natl. Acad. Sci. USA. 94, 10319-10323 (1997). expressing enhanced green fluorescent protein (EGFP) alone, plus 20 µg pCMVΔR8.2, and 10 µg of the heterologous VSV-G envelope construct pCVSV-G; all other vector constructs were gifts from I. Verma, Salk Institute for Biological Sciences, CA, USA) into 293T cells using methods previously described by Blomer et al (Blomer, U. et al. Highly efficient and sustained gene transfer in adult neurons with a lentivirus vector. J. Virol. 71, 6641-49 (1997). Briefly, pCMVΔR8.2, a CMV promoter-driven packaging plasmid; heterologous VSV-G envelope construct and HMD/ IN were transiently cotransfected into the packaging cell line 293T by calcium phosphate precipitation. During the next three days the transfected 293 T cells released retroviruses into the culture medium. The conditioned culture medium was collected at 24, 48 and 72 hours and stored in aliquots at -80°C.

### Viral Concentration

Retroviruses were filtered through a 0.45 µm pore size filter and concentrated by centrifugation at 50,000 g for 120 min. The titre of the virus was determined by transducing 293T cells and the number of EGFP positive cells was determined by counting of EGFP positive cells using a fluorescent microscope (Leica, Australia). Using an eyepiece graticule with a 10 x 10 grid covering an area of 0.0624 mm² at 400X magnification. Confluent cells in ten random fields were counted and the final result multiplied 1.6 to give the number of cells per mm². The titre (TU/ ml) = EGFP positive cells / mm² x flask (mm²) ÷ vectors(ml). This was further confirmed by by flow cytometry, which was performed on a FACScalibur flow cytometer (Becton Dickson, San Jose, CA). Data was analyzed using Cell-Quest software (Becton Dickinson, San Jose, CA, USA). Collected virus was tested for replication-competent viruses (replication competency) by assaying for rescue of integrated provirus that expressed EGFP.

The titer of the vector was also determined by an alternative more sensitive methodology. Experiments were performed in 293T cells adding serial dilutions of vector to 10⁵ cells/well in a 6-well plate. Transduced cells were analysed for EGFP expression by FACS and CellQuest (Becton Dickinson) software. This method of determining vector concentration provided a higher estimate of viral concentration compared to the manual counting method using the fluorescent microscope described above. For example, where viral titer was determined to be 4x10⁶ TDU using the manual method, when titered using the new FACS method the titer was estimated to be 5x10⁸ TDU.

Hence, where it is elsewhere in this specification described that, for example, male Wistar rats were transduced with a total amount of vector of 4 X 10⁶ transduction units (see "Transduction of hepatocytes in vivo"), it will be understood that that estimate was determined on the basis of the manual method and that, when determined by the FACS method, the same experiment would be stated as having infused a total amount of 5x10⁸ TDU. For the sake of clarity, and except where indicated otherwise, references to vector concentrations elsewhere in this specification are references to vector concentration as determined by the manual method described above. The skilled addressee will be aware, that determination of viral vector concentration by alternative methods can lead to alternative results and will thus be able to make suitable adjustments.

### Experimental Rats

Male Wistar rats, weighing 250 - 300g were housed in individual cages and exposed to 12 hr cycles of light and dark. Maintenance and experimental manipulation of male Wistar rats (250-300g body weight) were performed in accordance with the guidelines and regulations of the Australian Research Council on Animal Care. Royal North Shore Hospital/ University of Technology, Sydney, Animal Care and Ethics Committee approved all procedures.

### Transduction of hepatocytes in vitro

293T and Nit-1 cells (a transformed β-cell line derived from the nonobese diabetic mouse) were grown in Dulbecco's minimal essential medium (DMEM; Trace Biosciences, Australia) supplemented with 10% heat-inactivated fetal calf serum (FCS) (Trace Biosciences) at 37°C and 5% CO₂.

Primary rat hepatocytes were isolated using a two-step ethylenediaminetetraacetate (EDTA) collagenase digestion technique. The portal vein (PV) together with the abdominal aorta (AA) were cannulated and the liver flushed *in situ* with warmed (37°C) Hanks buffered salt solution without calcium and magnesium (HBBS H9394, Sigma, Australia), plus 1 mmol/L EDTA. This was followed by perfusion with 0.05% collagenase (80 µg/ ml) in Hanks balanced salt solution (HBSS) with calcium and magnesium (Sigma H9269). The digestion was neutralized with cold DMEM containing 10% FCS and the liver was removed. Using sterile technique, the liver capsule was removed; the cells were dispersed and allowed to pass through nylon mesh with a pore size of 250 µm. This was repeated using nylon mesh with a pore size of 100 µm. The hepatocyte fraction of the liver cells was purified using low speed centrifugation at 500 rpm for 2 minutes at 4°C in cold phosphate buffered saline (PBS). The centrifugation step was repeated three times, each time the medium supernatant was removed and the cell pellet resuspended in fresh PBS. Following the final wash cells were resuspended in DMEM supplemented with NaHCO₃ (44 mmol/L), insulin (100 mU/L), glutamic acid (2.5 mmol/L), proline (1 mmol/L), ascorbic acid (56 mg/L), penicillin (100 mg/ml), nicotinamide (10 mmol/L), epidermal growth factor (20 ng/ml), selenium salts (0.1 µg/ml) and 1 nmol/L hydrocortisone (modified DMEM). Cellular viability was determined by trypan blue exclusion. Preparations containing greater than 85% viable cells were plated at a density of 5 X 10⁵ cells/ per well into 6 well plates coated with Matrigel (available from BD Biosciences; obtained from C. Liddle, Westmead Hospital, Sydney, Australia) and cultured in modified DMEM. After 5 days, 1.5% DMSO together with CuSO₄ (2.5 µg/L), FeSO₄.7H₂ (834 µg/L) and ZnSO₄ (1.47 mg/L) were added to the medium for long-term maintenance of hepatocytes.

Although Matrigel strongly inhibits cell division primary hepatocytes were also irradiated 24 h after seeding. Irradiation was performed with a cesium-137 source at a dose rate of 75 cGy/ min to provide total doses of 0, 10, or 20 Gy. The effect of irradiation on DNA synthesis was assessed by [³H] thymidine incorporation (Ott, M., Stockert, R. J., Ma, Q., Gagandeep, S. & Gupta, S. Simultaneous up-regulation of viral receptor expression and DNA synthesis is required for increasing efficiency of retroviral hepatic gene transfer. J. Biol. Chem. 273, 11954-11961 (1998) over a 7 day period. The positive control was hepatocytes plated on rat tail collagen 1 (Collaborative Biomedical Products, Australia) (5 µg/cm²) which encourages cell growth. Hepatocytes were transduced with the pHR'CMVGFP lentiviral vector (5 x 10⁵ transducing units [TDU]/well) in the presence of 8 µg polybrene for 4 and 24 h after irradiation. Transduction efficiency was assessed 48 h and 7, 14, 21 and 28 days after vector exposure by fluorescence microscopy. Additionally, numbers of EGFP-positive cells were quantified by flow cytometry at 2 weeks and one month. EGFP-expressing cells were also fixed with 1% glutaraldehyde in phosphate buffered saline (PBS) and stained for the liver-specific marker glucose-6-phosphatase and quantitation was done by light microscopy.

### Transduction of hepatocytes in vivo

Male Wistar rats were divided into 4 groups of 16 animals. For group 1, the liver was segregated from the normal blood supply by ligating the right adrenal vein and successively clamping the infrahepatic vena cava, hepatic artery, portal vein and suprahepatic vena cava and the pHR'CMVGFP lentiviral vector (1.5 ml) was infused through a cannula inserted in the portal vein. During this procedure there was no blood supply to the liver for five minutes and then the clamps were removed for two minutes to allow blood flow to the liver. This procedure of intervallic infusion in full flow occlusion (FFO) was repeated three times. The total amount of vector infused was 4 X 10⁶ transduction units. Animals in group 2 were treated similarly; except they were infused with 0.9% saline. A 70% partial hepatectomy was performed on animals in group 3 by resecting the left lateral and median liver lobes. The right adrenal vein was ligated and 24 h later, vector was infused by the procedure detailed group 1. A fourth group of animals was treated the same as group 3, except these animals were infused with 0.9% saline.

Livers from all groups were harvested at 1, 2 and 8 weeks and at 6 months. EGFP expression was assessed in cryosections of liver using a rabbit horseradish peroxidase conjugated anti-EGFP antibody (Clontech, Australia). The efficiency of hepatocyte transduction was determined by randomly scoring 400 hepatocytes surrounding the portal spaces and interlobular areas and data was expressed as a percentage of EGFP-positive hepatocytes.

Analysis in live cells was performed by laser-scanning confocal microscopy. For confocal microscopy the liver cell preparation was prepared as previously described and was viewed immediately at room temperature in a Leica TCSNT Confocal system using an upright DMRBE microscope and Leica Plan fluotar objective lens 40x oil, N.A.1.00-0.50/WD 0.08. Software used was TCSNT version 1.6.587, excitation with 488 nm line from an Ar/Kr laser and DP 488/568, emission collected using 8 accumulations with BP525/550, glowover. Objective lens was Leica Plan fluotar 40x oil, N.A.1.00-0.50/WD 0.08. The laser settings were not changed during the course of an experiment, allowing control preparations (without insulin-GFP) and transfected preparations (with insulin-GFP) to be compared.

To determine whether this surgical technique induced damage to the liver, as evidenced by cell proliferation, 5-Bromo-2'-deoxyuridine (BrDU, Sigma, MO, USA) was dissolved in PBS (30 mg/ ml) and injected intraperitoneally at 18, 28, and 48 h after surgery (partial hepatectomy or sham-operated) and into untreated animals (3 animals per timepoint). Rats were killed 2 h after BrDU delivery. Livers were excised, fixed in neutral buffered formalin, processed by conventional histological techniques, and 5 µm sections were cut. BrDU incorporation was determined by immunohistochemistry. The labeling index was calculated as the percent BrDU-positive hepatocyte nuclei after observing at least 4000 cells from randomly selected fields in each sample.

### Insulin gene delivery in STZ - induced diabetic rat liver

To determine if *in vivo* transduction of hepatocytes with HMD/ INS-FUR could correct diabetes rats were made diabetic by peritoneal injections of streptozotocin (STZ; Sapphire Bioscience, Australia) dissolved in acetate buffer, pH 5.2 at a rate of 75mg/kg of body weight. The diagnosis of diabetes was based on 3 or more consecutive, random blood glucose levels of more than 14mmol/L. Blood glucose was measured by blood glucose electrodes (Medisense Australia) using tail blood. The rats were divided into three groups. The HMD/INS-FUR vector was administered into Group 1 animals (n = 10) with 4 x 10⁶ TU and Group 2 animals (n = 6) with empty vector HMD (4 x 10⁶ TU) via the portal vein as described herein. Group 3 (n = 10) consisted of normal non diabetic rats as the control. Following delivery of the insulin gene or empty vector, the rats were monitored for changes in body weight and blood glucose levels regularly: daily for the first 2 weeks and then bi-weekly subsequently. Animals were sacrificed at 2 months and 500 days for analysis (n = 5). Some of the animals were sacrificed at 60 days, this was all the empty vector animals, one died before this and the results from this animal are not included. Five of the normal animals and 5 or the HMD/ INS-FUR transduced animals were sacrificed at 60 days and the rest kept for 500 days.

### Functional studies

### (a) IVGTT

Intravenous glucose tolerance tests (IVGTT) were performed after an overnight fast on all rats. At various times (0, 2, 5, 10, 15, 30, 60 and 120 minutes) after the infusion of glucose (0.5g/kg body weight) blood samples were collected from the tail vein and assayed for glucose, c-peptide and insulin.

### (b) Radioimmunoassay

Insulin and c-peptide levels were determined by radioimmunoassay (RIA) after tissues were excised, weighed, homogenised and incubated in 0.18N HCL in 70% ethanol for 18 hours at 4°C. For measurement of insulin content, samples were diluted before being assayed as previously described using specific rat or human standards (Tuch, B. E. et al. Function of a genetically modified human liver cell line that stores, processes and secretes insulin. Gene Ther. 10, 490-503 (2003)). Results were compared to the wet weight of the tissue. For gel filtration chromatography, ethanol was evaporated, samples lyophilized and then reconstituted in column buffer (Short, D. K., Okada, S., Yamauchi, K. & Pessin, J. E. Adenovirus-mediated transfer of a modified human proinsulin gene reverses hyperglycaemia in diabetic mice. Am J Physiol; 275: E748-E756 (1998)). Insulin in serum samples and column fractions was measured using an ultrasensitive RIA for human insulin (Linco Research, MO, USA), which has less than 1% cross-reactivity with rodent insulin and 6% cross reactivity with human proinsulin. Insulin was resolved from proinsulin by gel filtration chromatography on a 1.0 x 120 cm Sephadex G-50 Super Fine column (Pharmacia) as described previously (Short *et al,* 1998).

A commercial RIA (Eurodiagnostica, Malmö, Sweden) was used to measure levels of human c-peptide. Rat c-peptide was determined by a commercial kit (Linco, MO, USA) using rat c-peptide standards (Akpan, J. O., Weide, L. G. & Gingerich, C. W. A specific and sensitive radioimmunoassay for rat c-peptide. Int. J. Pancreatol. 13, 87-95 (1993).

To assess liver function, aspartate aminotransferase (AST) or alanine aminotransferase (ALT) were measured throughout the study using commercial kits (Roche Diagnostics, Switzerland) and analyses using a microplate reader (Bio-Rad) in kinetic mode.

### RT - PCR analysis

Liver, pancreas, spleen, kidney and lung were harvested at 2 months after vector infusion and at the experimental endpoint (500 days), flash frozen in liquid nitrogen and stored at -180°C. Total RNA was extracted using a RNeasy Protect Mini Kit (QIAGEN) or Trizol (Invitrogen, CA, USA) and treated with RNAlater (Qiagen Australia) to prevent degradation. To eliminate genomic DNA contamination the samples were treated with DNase I (Invitrogen, CA, USA). Real time quantitative PCR was conducted using SYBR green I (Applied Biosystems, CT, USA) using an ABI Prism 7900 HT Sequence Detection System (Applied Biosystems CT, USA). The optimal forward and reverse primer concentrations were determined for PDX-1, NeuroD, Ngn 3 and β-action by titration. The amplification efficiencies for each primer set were also determined by varying cDNA template concentration and were approximately equal for each data set. Data was presented as differences between lowest threshold values (ΔC(t)), for the transcripts of interest and the internal standard, β-actin. The PCR products were separated on 2.5% agarose gels.

2µg total RNA was denatured at 65°C for 5 min using Omniscipt RT kit (QIAGEN) and random primers (Promega) for reverse transcription. For cDNA synthesis, the PCR reaction mixture included PCR buffer 22.5 µl (Promega), 25mM dNTPs 1.8 µl, Tac polymerase (Promega) 1.8 µl, and 6.8 µg of each primer for reaction. Total volume of 25 µl for 35 cycles. PCR parameters that consisted of denaturing at 94°C for 30 sec, annealing at 64 °C for 30 sec, and elongating at 72 °C for 1.30 min.

For the following primers annealing was at 64 °C for 30 sec. EGFP (77 bp), forward: 5'-CAACAGCCACAACGTCTATATCATG-3' (SEQ ID NO:1); reverse: 5'-ATGTTGTGGCGGATCTTGAAG-3' (SEQ ID NO:2); INS-FUR (248 bp), forward: 5'-AGCCTTTGTGAACCAACACC-3' (SEQ ID NO:3), reverse: 5'-CCAGTTGGTAGAGGGAGCAG-3' (SEQ ID NO:4). All other primers are specific for the rat sequence. Ngn3 (255 bp), forward: 5'-AGAAAGGGAGGGAGTCAGAG-3' (SEQ ID NO:5), reverse: 5'-CACCAGGAAGTATGGGAGTC-3' (SEQ ID NO:6). PDX1 (243 bp), forward: 5'-AAACGCCACACAAGGAGAAC-3' (SEQ ID NO:7), reverse: 5'-CTGTTATGGGACCGCTCAAG-3' (SEQ ID NO:8); NeuroD/Beta2 (300 bp), forward: 5'-GGACTTTCTTGCCTGAGCAGA-3' (SEQ ID NO:9), reverse: 5'-AACTCGGTGGATGGTTCGTGT-3' (SEQ ID NO:10).

For the following primers annealing was at 65 °C for 2 min. Insulin I (331 bp), forward: 5'-ATGGCCCTGTGGATGCGCTT-3' (SEQ ID NO:11), reverse: 5'-TAGTTGCAGTAGTTCTCCAGCT-3' (SEQ ID NO:12); insulin II (209 bp), forward: 5'-ATGGCCCGTGGGATCCGCTT-3' (SEQ ID NO:13), reverse: 5'-TGCCAAGGTCTGAAGGTCAC-3' (SEQ ID NO:14); somatostatin (187 bp), forward: 5'-GTTTCTGCAGAAGTCTCTGG-3' (SEQ ID NO:15), reverse: 5'-AGTTCTTGCAGCCAGCTTTG-3' (SEQ ID NO:16); glucagon (210 bp), forward: 5'-GACCGTTTACGTGGCTGG-3' (SEQ ID NO:17), reverse: 5'-CGGTTCCTCTTGGTGTTCATCAAC-3' (SEQ ID NO:18); pancreatic polypeptide (214 bp), forward: 5'-TGAACAGAGGGCTCAATACGAAAC-3' (SEQ ID NO:19), reverse: 5'AGACAGAAGGGAGGCTACAAATCC-3' (SEQ ID NO:20); NKX 2.2 (188 bp), forward: 5'-CACGCAGGTCAAGATCTG-3' (SEQ ID NO:21), reverse: 5'-TGCCCGCCTGGAAGGTGGCG-3' (SEQ ID NO:22); NKX 6.1 (205 bp), forward: 5'-ATGGGAAGAGAAAACACACCAGAC-3' (SEQ ID NO:23), reverse: 5'-TAATCGTCGTCGTCCTCCTCGTTC-3' (SEQ ID NO:24), Pax 4 (214 bp), forward: 5'-TGGCTTTCTGTCCCTTCTGTGAGG-3' (SEQ ID NO:25), reverse: 5'-TCCAAGACTCCTGTGCGGTAGTAG-3' (SEQ ID NO:26), Pax 6 (545 bp), forward: 5'-AAGAGTGGCGACTCCAGAAGTTG-3' (SEQ ID NO:27), reverse: 5'-ACCACACCTGTATCCTTGCTTCAGG-3' (SEQ ID NO:28); PC2 (359 bp), forward: 5'-GACTGGTTCAACAGCCATGG-3' (SEQ ID NO:38), reverse: 5'-TAGCCGTCACAGTTGCAGTC-3' (SEQ ID NO:39); GLUT 2 (304 bp), forward: 5'-TTAGCAACTGGGTCTG-3' (SEQ ID NO:29), reverse: 5'-GGTGTAGTCCTACACTCATG-3' (SEQ ID NO:30); glucokinase (136 bp), forward: 5'-AAGGGAACAACATCGTAGGA-3' (SEQ ID NO:31), reverse: 5'-CATTGGCGGTCTTCATAGTA-3' (SEQ ID NO:32); β-actin (349 bp), forward: 5'-CGTAAAGACCTCTATGCCAA-3' (SEQ ID NO:33), reverse: 5'-AGCCATGCCAAATGTCTCAT-3' (SEQ ID NO:34). For the p48 primer (285 bp) annealing was at 60 °C for 1.5 min, forward: 5'-GTCCCTGGAGCATTTTCCCG-3' (SEQ ID NO:35), reverse: CTGAGGAACTCTTACCTCCGC-3' (SEQ ID NO:36). The products were separated on 2.5% agarose gels by electrophoresis.

### Immunohistochemical Analysis

Liver and pancreas tissues were collected 2 months after vector infusion and at the end of the experiment (500 days) and fixed in 10% buffered formalin and embedded in paraffin and sections cut at 5 microns and mounted onto slides. The slides were deparaffinised by xylene and hydrated through a graded series of alcohols. The endogenous peroxidase was inactivated by treatment of 3% hydrogen peroxide. Prior to antibody labeling, 1% BSA was applied to the slides to prevent non-specific staining.

Using vector ABC kit (Burlingame, USA) the slides were incubated with the primary antibody for 1hr. the sections were washed with PBS for 5 min prior to 1 hr incubation with biotinylated secondary antibody. The slides were washed again for 5 minutes in PBS buffer. The sections were then incubated for 30 min with vectostain ABC reagent. The slides were then washed again for 5 min. in PBS. The presence of islet hormones was determined by immunoperoxidase immunohistochemistry using diaminobenzidine (DAB) as substrate. Primary antibodies against insulin (mouse anti-human insulin monoclonal antibody1:300; BioGenex, MU 029-UC, USA), glucagon (goat anti-glucagon polyclonal antibody, SC-7780; 1:200; Santa Cruz CA, USA), and somatostatin (goat anti somatostatin polyclonal antibody; 1:200; SC-7819, Santa Cruz CA, USA) were used. All cells in ten random fields were scored and was expressed as the number of positive cells per mm² of tissue.

### Western blot analysis

Rat liver and pancreas were homogenized in a sonicator. Nuclear and cytoplasmic extracts were prepared using a Nuclear Extraction Kit (ChemiconTemecula, USA). 1 µg of tissue extracts were fractionated by SDS - PAGE, protein was transferred to nitrocellulose and blocked for 60 min in 3% BSA. The blot was incubated overnight at 4°C with primary antibody. The bands visualized using ECL Western blotting analysis system (Amersham, UK) for detection of immobilized specific antigens. The primary antibody used for PDX-1(rabbit anti-PDX1 polyclonal antibody, 1:5000; Chemicon, USA) detects a major band at 43 kD. The primary antibody used for Neuro D (1:5000; Neuro D N-19: sc-1084; Santa Cruz CA, USA) detects a major band at 50 kDa.

### Statistical analysis

Data were analyzed with the computer statistical package NCSS (Hintze, J. L. Number cruncher statistical system: version 5.01. Kaysville, Utah, 1991). Differences between groups were determined by Student's paired t test or, if there were more than two groups, by one-way analysis of variance after log transformation of the data.

### Electron Microscopy

For morphological analysis by electron microscopy liver tissue was fixed and processed according to conventional techniques using uranyl acetate block staining and Reynold's lead citrate counter-staining of ultrathin sections (80nm) as follows. 1 mm³ fragments of tissue from the transduced rat livers were fixed in 2% glutaraldehyde & 1% paraformaldehyde for 1 hour at 4°C in 0.1M Cacodylate buffer at pH 7.4, followed by 1% osmium tetroxide for 1 hour at 4°C. A block stain was carried out in aqueous 2% uranyl acetate at room temperature for 1 hour. Tissue was dehydrated in solutions of graded alcohol and embedded in Durcupan (Fluka, Switzerland). Ultra-thin sections (80 nm) were cut on a Reichert Ultracut-S and counter-stained with Reynold's lead citrate. Stained sections were examined at 80 kV on a Phillips CM10 transmission electron microscope. Granule size was measured directly from the images after calibrating with replicas.

For immunoelectronmicroscopy, a post-embedding immunogold procedure was used to localize intracellular insulin. Single cell suspensions of control Nit 1 β cells (mouse pancreatic β-cell line, used as a positive control) and fixed for 2 hours at room temperature in 4% formalin in 0.1M phosphate buffer containing 40 mM betaine, pH 7.4. Cells were allowed to settle between washes of phosphate buffered saline, 0.9% sodium chloride, ethanol series to 70% followed by embedding in freshly-made LR White. Cells were centrifuged gently at 0.2 rcf for 10 min. in a MiniSpin Plus Eppendorf centrifuge for the last embedding step only. Tissue was treated similarly without the necessity to spin down the material. Hardening was at 48°C for 48 hours. Sections were cut using a MT-1 microtome and labeling procedures were carried out immediately after sectioning as described by Saccomano et al ("Immunoelectron microscopy: a new method for detection of insulin antibodies", J Histochem Cytochem 1993; 41: 1233-1239), with particular details as follows: non specific binding was blocked using 1% bovine serum albumin and 1% glycine in phosphate buffered saline at room temperature for 2 hours. A goat antimouse IgG [EMGMHL 10] 10nm gold probe 1:50 or 1:25, BBInternational, Australian Laboratory Services Pty Ltd, Sydney) incubated for 2 hours at room temperature was directed against a monoclonal mouse anti-human insulin (1:70 or 1:25) primary antibody (BioGenex, San Ramon, CA, USA) incubated overnight at 4°C. Primary antibody was replaced by PBS to assess the level of non specific binding of the gold probe. Sections were stained with 1.5% aqueous uranyl acetate alone for 9 or 15 minutes followed by distilled water wash before observing at 80kV in a JEOL JEM 1010 transmission electron microscope. Lead citrate staining was not performed to avoid confusion between possible lead precipitates and gold particles.

### Surgical Procedure

The surgical procedure is schematically illustrated in **Figure 1****.** Following ligation of the right adrenal vein the veins and arteries were clamped in the following order: infrahepatic vena cava, hepatic artery, portal vein, suprahepatic vena cava. **Figure 2** illustrates the liver before and after draining during the procedure. The lentiviral vector HMD/INS-FUR containing furin-cleavable human proinsulin cDNA (FurHPI) is schematically illustrated in **Figure 3a****.** Vector was infused into the liver, via a cannula inserted in the portal vein, in a sterile saline solution 4ml at a titre of 4x10⁶ transduction units and at a rate of 15-20 sec/ ml from a syringe. During infusion the liver was without blood for 5 minutes. The vessels were then released in the following order: suprahepatic vena cava, infrahepatic vena cava, portal vein, hepatic artery, for two minutes to allow the liver to be bathed in blood.

The procedure of clamping-infusion and release was then repeated twice. That is, the vessels were re-clamped in the initial order: infrahepatic vena cava, hepatic artery, portal vein, suprahepatic vena cava; the infusion repeated as above; the vessels released in the order suprahepatic vena cava, infrahepatic vena cava, portal vein, hepatic artery, for two minutes to allow the liver to be bathed in blood; re-clamped again in the initial order: infrahepatic vena cava, hepatic artery, portal vein, suprahepatic vena cava; and the infusion repeated once more. In total three infusions were carried out.

After completion of the last infusion the vessels were released in the order: suprahepatic vena cava, infrahepatic vena cava, portal vein, hepatic artery.

### Example 2

### pHR'CMV and enhanced green fluorescent protein

Using the lentiviral system pHR'CMV, vectors were constructed as described herein which expressed the enhanced green fluorescent protein (EGFP) to transduce primary rat hepatocytes in culture with a resulting 60% transduction of non-dividing cells. The expression of EGFP was evident from 24 hours and was persistent for one month.

For the *in vivo* delivery of EGFP to adult Wistar rats, using the portal infusion technique described herein, three weeks after infusion FACS sorting and confocal microscopy revealed 90% transduction of primary hepatocytes. Our *in vivo* transductions resulted in stable expression of EGFP for 9 months in animal livers.

### Example 3: Delivery of insulin-expressing vectors to Nonobese Diabetic (NOD) mice:

### Materials and Methods

Vector construction, generation of virus and viral concentration is as described in Example 1.

### Insulin gene delivery to diabetic NOD mice

Female NOD mice, weighing 20-25 g were housed in individual cages and exposed to 12 hr cycles of light and dark. Royal North Shore Hospital/ University of Technology, Sydney, Animal Care and Ethics Committee approved all procedures. The mice spontaneously developed diabetes between 12-20 weeks of age. The diagnosis of diabetes was based on 3 or more consecutive, random blood glucose levels between 11-13 mmol/L. Blood glucose was measured by blood glucose electrodes (Medisense Australia) using tail blood. The rats were divided into three groups. The HMD/INS-FUR vector was administered into Group 1 animals (n = 3) with 1.4-2.5 x 10⁵ TU and Group 2 animals (n = 3) with empty vector (1.4-2.5 x 10⁵ TU) via the portal vein as described herein. Following delivery of the insulin gene or empty vector, the mice were monitored for changes in body weight and blood glucose levels regularly. Group 3 (n = 3) consisted of diabetic mice as the control.

### IVGTT

An intravenous glucose tolerance test (IVGTT) was performed after an overnight fast on all mice. At various times (0, 2, 5, 10, 15, 30, and 50 minutes) after the infusion of glucose (0.5g/kg body weight) blood samples were collected from a cannula and assayed for glucose and insulin.

### Immunohistochemical Analysis

Liver and pancreas tissues were collected 2 months after vector infusion and fixed in 10% buffered formalin and embedded in paraffin and sections cut at 5 microns and mounted onto slides. The slides were deparaffinised by xylene and hydrated through a graded series of alcohols. The endogenous peroxidase was inactivated by treatment of 3% hydrogen peroxide. Prior to antibody labelling, 1% BSA was applied to the slides to prevent non-specific staining.

Using vector ABC kit (Burlingame, USA) the slides were incubated with the primary antibody for 1hr the sections were washed with PBS for 5 min prior to 1 hr incubation with biotinylated secondary antibody. Then the slides were washed again for 5 minutes in PBS buffer. The sections were then incubated for 30 min with vectostain ABC reagent. The slides were then washed again for 5 min in PBS. The sections were then incubated in DAB, rinsed with water and mounted. The antibody was: mouse anti human insulin monoclonal antibody (1:300) from BioGenex (USA).

### Surgical Procedure

The surgical procedure is schematically illustrated in **Figure 1****.** Following ligation of the right adrenal vein the veins and arteries were clamped in the following order: infrahepatic vena cava, hepatic artery, portal vein, suprahepatic vena cava. The lentiviral vector HMD/INS-FUR containing furin-cleavable human proinsulin cDNA (FurHPI) is schematically illustrated in **Figure 3a****.** Vector was infused into the liver, via a cannula inserted in the portal vein, in a sterile saline solution 0.3-0.5 ml at a titre of 1.4-2.5 X 10⁵ transduction units and at a rate of 15-20 sec/ ml from a syringe. During infusion the liver was without blood for 3-4 minutes. The vessels were then released in the following order: suprahepatic vena cava, infrahepatic vena cava, portal vein, hepatic artery, for one to two minutes to allow the liver to be bathed in blood.

The procedure of clamping-infusion and release was then repeated once or twice. That is, the vessels were re-clamped in the initial order: infrahepatic vena cava, hepatic artery, portal vein, suprahepatic vena cava; the infusion repeated as above; the vessels released in the order suprahepatic vena cava, infrahepatic vena cava, portal vein, hepatic artery, for two minutes to allow the liver to be bathed in blood; re-clamped again in the initial order: infrahepatic vena cava, hepatic artery, portal vein, suprahepatic vena cava; and the infusion repeated once more in some cases. In total two or three infusions were carried out.

After completion of the last infusion the vessels were released in the order: suprahepatic vena cava, infrahepatic vena cava, portal vein, hepatic artery.

### Results and Discussion

### Efficient transfection of hepatocytes

Isolation of hepatocytes by the dual perfusion of the PV and AA resulted in increased cell viability as compared to perfusion of the PV alone (85.7 ± 6.2% versus 67.5 ± 7.8%, respectively, p, 0.03) as determined by trypan blue exclusion. The cells were morphologically normal as assessed by both light and electron microscopy (data not shown), with maintenance of gap junctions between cells and limited vacuolation.

Primary hepatocytes are difficult to maintain in culture according to known procedures. Hepatocytes grown on Matrigel in the medium optimised in this study, with or without irradiation, did not undergo cell division, as assessed by [³H] thymidine uptake following seven days in culture. Cells grown on collagen reached a maximum growth rate at day two and this plateaued between days five to six (data not shown). The capacity of lentiviral particles to transduce the cultured hepatocytes was determined by both fluorescence microscopy and flow cytometry. EGFP expression was seen as early as 48 h post-transduction and persisted for at least 1 month in culture **(****Figure 4a****).** Up to 87% ± 1.2 (n=3) of the irradiated cells were positive for EGFP expression as determined by flow cytometry **(****Figure 4b****).** The EGFP positive cells were also positive for glucose-6-phosphatase, which further confirms their hepatocyte lineage (data not shown). Growth of hepatocytes on collagen did not increase transduction efficiency.

The lentiviral vector was delivered to the liver of diabetic rats by FFO and the transduction efficiency compared in non-hepatectomised and partially hepatectomized animals. The total wet weights of liver were compared 48 h after partial hepatectomy to liver weights of control rats and were found to be not significantly different (NSD) (14.30 ± 0.4 g and 14.7 ± 0.8 g, respectively, n=3). To determine if FFO stimulated cell division of hepatocytes, the number of hepatocytes that were proliferating at various times after either FFO or partial hepatectomy was assessed by BrDU incorporation. In control rats 0.1 ± 0.06% (n=3) hepatocytes were proliferating at 18h and this rate did not significantly alter at any time point. At 18 h in animals that had undergone FFO, 0.3 ± 0.2% hepatocytes were proliferating, compared to 0.4 ± 0.3% at 28 h and 0.1 ± 0.04% at 48 h (n=3). These values did not differ significantly from those recorded for control animals. By comparison, in animals that had undergone a partial hepatectomy 12.8 ± 2.4% hepatocytes were proliferating at 18 h. This value increased to 21.4 ± 4.3% at 28 h and then decreased to 7.4 ± 1.2% at 48 h.

To determine the transduction efficiency, liver cells were isolated after transduction at 48h, 1, 2, 4, 8 weeks and 6 months and were assessed for GFP-positivity by fluorescence microscopy, immunohistochemistry using anti-GFP antibodies, and directly in viable cells by confocal microscopy **(****Figure 4c****).** Maximal GFP-gene expression was detected 2 weeks after transduction as determined by both light and confocal microscopy (75.0 ± 5%; n=4) and this level of expression was maintained for 6 months (74.5 ± 4.8%; n=4). In animals that had undergone a partial hepatectomy prior to transduction with the vector by FFO, there was no significant difference in the number of EGFP-expressing hepatocytes (76.0 ± 5%) (n=4) after 6 months compared to non-hepatectomised animals.

As can be seen from **Figure 5B** and **Figure 7** a large number of the cells in the liver of the animals transduced with the viral vector containing the furin-cleavable insulin cassette have been converted into cells that stain positively for insulin. There was also some evidence of pancreatic transdifferentiation as glucagon and somatostatin staining was also detected **(****Figure 6****)** in liver tissue that was transduced with the HMD/INS-FUR construct, but no pancreatic hormones were detected in tissue transduced with the empty vector **(****Figures 5 and 6****).** Staining for insulin was 4.8-fold higher than glucagon and 11.2-fold higher than somatostatin **(****Figure 7****).** Unlike other studies that have engineered β (beta) cell neogenesis from liver cells (Ber et al "Functional, persistent, and extended liver to pancreas transdifferentiation" J. Biol. Chem. 2003, 278:31950-7; Kojima et al "NeuroD-betacellulin gene therapy induces islet neogenesis in the liver and reverses diabetes in mice" Nat. Med. 2003, 9:596-603), the insulin-containing cells are not isolated to the area surrounding the portal circulation, but are scattered throughout the liver tissue **(****Figure 5B****).**

### HMD/ INS-FUR transduction stimulated expression of pancreatic hormones in liver

Numbers and distribution of pancreatic hormone-producing cells in the livers of rats transfected with lentiviral vector were determined after 2 months. **Figure 8a** shows extensive staining for insulin in normal pancreatic islets from a nondiabetic animal. By comparison pancreatic tissue from STZ-diabetic animals that were subsequently transfected with the HMS/INS-FUR vector, which show few insulin-positive cells (1.5 ± 1.4, n=20) **(****Figure 8b****).** Following transduction with HMD/INS-FUR, a large number of hepatocytes were converted into insulin-positive cells **(****Figure 8d****).** There was also evidence of some pancreatic transdifferentiation as glucagon and somatostatin staining was also detected **(****Figure 8e****-d).** No pancreatic hormones were detected in tissue isolated from animals injected with the empty vector or normal control animals.

Electron microscopy studies revealed that granules (270-330nm in diameter) were scattered throughout the cytoplasm of rat livers treated with HMD/INS-FUR **(****Figure 8g****).** Immunoelectron micrographs **(****Figure 8h****)** of insulin producing cells in the liver of a STZ-diabetic Wistar rat transduced with the lentiviral vector HMD/INS-FUR are identical to those observed in the β-cell line, Nit-1 **(****Figure 8i****).** No evidence of inflammatory cell infiltration or necrosis of the liver was observed in these animals. There were no granules detected in rats treated with HMD alone or in control animals.

### HMD/ INS-FUR transduction induced hepatic insulin production

In one experiment insulin storage following acid/ethanol extraction was 24,500.93 ± 1,064.2 ng/ liver (n=3) in HMD/ INS-FUR-treated animals, compared to 61,285 ± 1235.4 ng recorded in normal rat pancreas. By contrast, STZ-treated rat pancreas contained over 40-fold less insulin than normal pancreas (1,500 ± 645.2 ng).

Sephadex G-50 gel filtration chromatography was used to separate insulin from its precursor proinsulin isolated from livers of animals treated with HMD/INS-FUR. This yielded two distinct peaks, the first of which represented proinsulin and partially processed material while the second larger peak corresponded to fully processed insulin **(****Figure 9****).**

In one experiment insulin storage following acid/ethanol extraction was 17,625.8 ± 2.1 ng (n=3) per liver. For comparison, the amount in the pancreas of a non-diabetic rat was 61,000 ng. This is 35%-40% the insulin content, considerably higher than Kojima *et al* (2003, *ibid*) who found mouse livers transduced by adenoviral vectors which expressed NeuroD and betacellulin stored 20% of a non-diabetic mouse pancreas and Ber *et al* (2003, *ibid*) who found mouse livers transduced by adenoviral vectors expressing PDX-1 stored 1-3% of the insulin in non-diabetic mouse pancreas. In human islet transplantation, the minimum number of islets required for insulin independence is about 9,000 islet equivalents per kg, or 600,000 islets for a 70 kg person. This corresponds to 30-50% the number of islets in the pancreas of a non-diabetic individual (Ryan et al "Successful islet transplantation. Continued insulin reverse provides long-term glycemic control" Diabetes 2002, 51:2148-57). In islet transplantation all the islets do not survive, so the insulin content observed in the present methods is well within the acceptable limits for insulin independence.

Reverse transcription-polymerase chain reaction (RT-PCR) analysis indicated that there was no evidence of insulin expression in other body tissues, indicating that insulin expression was specific and isolated to the liver. (see **Figure 10**).

### Reversal of diabetes in STZ-diabetic rats

The diabetic state of experimental animals was assessed by determining blood glucose levels. **Figure 11a** shows that blood glucose levels of STZ-diabetic Wistar rats fell rapidly to subnormal levels immediately after transduction with HMD/INS-FUR vector. By day 5, blood glucose levels of insulin-transfected animals were not significantly different to non-diabetic controls and reversal of diabetes was maintained for 500 days. STZ-diabetic rats treated with HMD alone remained hyperglycemic until their sacrifice at day 60. The body weights of rats treated with HMD/INS-FUR were not significantly different to those of non-diabetic controls until day 125, they were however lower (P<0.05) between days 135 and 275, but were once again not significantly different from day 300 onwards **(****Figure 11b****)**. The body weights of rats treated with the empty vector alone continued to fall from day 50 until sacrifice at day 60, at which time they were significantly lower (P<0.01) than both nondiabetic controls and animals treated with the HMD/INS-FUR vector. There was no elevation of AST and ALT levels in animals treated with HMD/INS-FUR compared to control animals throughout the entire period of the experiment (data not shown). Thus, this methodology of vector delivery does not induce significant amounts of liver damage. Blood glucose levels remained within the normal range for the duration of the observation period, 500 days. STZ-diabetic rats treated with the vector alone remained diabetic until sacrifice at day 60. These results are consistent with reversal of diabetes in experimental animals within five days of administering the vector and with stable maintenance of that reversal over 500 days with no evidence of autoimmune attack.

### HMD/ INS-FUR transduced animals displayed regulated insulin secretion

The responsiveness of experimental animals to increased serum glucose was assessed using glucose tolerance tests. After an overnight fast non-diabetic control rats and STZ-diabetic rats treated with vector containing the insulin cassette were given an intravenous dose of glucose (0.5 g/ kg body weight). Blood glucose levels were measured over 120 min. There was no significant difference between the ability of animals transduced with the HMD/INS-FUR vector and normal control animals to normalize blood glucose levels after delivery of a bolus intravenous glucose dose **(****Figure 12a****),** indicating that our transfection procedure resulted in regulated insulin secretion in response to glucose stimuli. Levels of human c-peptide and human insulin in serum samples taken during the IVGTT from HMD/INS-FUR treated animals **(****Figures 12b****-c)** yielded similar results to those recorded for normal animals. Negligible levels of rat c-peptide were detected in serum from STZ-diabetic HMD/INS-FUR treated animals (data not shown). These results are consistent with normal glucose-responsiveness in the experimental animals.

The stability of the reversal of diabetes (over 500 days) is also consistent with the absence of autoimmune destruction of the insulin-expressing cells.

Normal liver function was assessed by determination of albumin, aspartate transaminase and alanine aminotransferase levels in experimental animals. All were normal in animals transduced to express insulin, indicating that the transduction and expression of insulin does not adversely affect normal liver function.

### HMD/ INS-FUR transduction stimulated expression of β-cell transcription factors

Tissue distribution of insulin expression following FFO delivery of the insulin gene to liver was determined. EGFP expression was isolated to the livers of pHR'CMV/GFP treated rats **(****Figure 13a****).** Similarly, INS-FUR expression was restricted to the livers of HMD/INS-FUR treated rats. EGFP and insulin transcripts were not detected in livers from control animals and animals injected with empty vector alone (results not shown). **Figure 13b** shows that the transduction of STZ-diabetic rats with the HMD/INS-FUR lentiviral vector induced the expression of several pancreatic transcription factors and expression was maintained over the 500 days of the experiment. As expected, normal liver was negative for all pancreatic transcription factors examined, while normal pancreas was positive. Unexpectedly, empty vector transduced livers expressed both PDX-1 and NeuroD. However, western analysis **(****Figure 13c****)** revealed expression of both these transcription factors in liver tissue transduced with the HMD/INS-FUR vector and no expression was detectable in tissue transduced with the empty vector alone. Other transcription factors, which were positive in HMD/INS-FUR, transduced livers, were Ngn3, Nkx 2.2 and Nkx 6.1. The expression of glucagon and somatostatin was detected in rat liver transduced with HMD/INS-FUR alone and this observation was confirmed by immunohistochemical analysis **(****Figure 8e****-f),** but pancreatic polypeptide was not expressed. The transduction of diabetic animals with HMD/INS-FUR did not result in complete pancreatic transdifferentiation of the liver tissue as rat insulin 1 and 2, insulin proconvertase PC2 and the exocrine marker P48 were not detected **(****Figure 13b****).** Quantitative real time PCR analysis of the β cell transcription factors PDX-1 (4.3 ± 0.9), NeuroD (5.7 ± 1.1) and Ngn 3 (7.4 ± 1.8) levels in HMD/ INS-FUR treated livers sampled at 500 days indicated that they were all significantly (P< 0.001) lower than those expressed in normal pancreatic tissue (PDX-1: 12.5 ± 2.3, NeuroD: 16.8 ± 1.4, Ngn3: 18.5 ± 2.2).

### Reversal of diabetes in NOD mice

**Figure 14** indicates that reversal of diabetes is seen almost immediately in NOD mice transduced with the HMD/INS-FUR vector and normal blood glucose was maintained for 2 months, when the animals were sacrificed. By comparison the blood glucose levels of untransduced diabetic control animals and animals transduced with the empty vector continued to increase to above 25 mM over one week to 10 days and were sacrificed at these earlier time points (results not shown).

**Figure 15** illustrates the results of an intravenous glucose tolerance test following a 16 hour (960 min) fast. It can be seen that the blood glucose levels of animals transduced with the HMD/INS-FUR vector returned to normal within 30 min, whilst the blood glucose of diabetic control animals remained at diabetic levels throughout. By comparison non-diabetic control animals with blood glucose levels between 5-6 mM took 50 minutes to return to normal. This was most likely due to the fact that whilst these animals exhibit normal blood glucose, the autoimmune destruction of the pancreatic islets has commenced and control over blood glucose is not truly normal.

**Figure 16a** illustrates the pancreas of an NOD mouse before the onset of diabetes at approximately 6 weeks of age, showing a significant number of insulin-containing cells and limited lymphocytic infiltration. By contrast **Figure 16b** is a section of a pancreas from an NOD mouse with blood glucose >11 mM, showing no appreciable positive staining for insulin and significant lymphocytic infiltration of the islets. This is typical of all pancreatic tissue of animals used for transduction. No indication of pancreatic regeneration was seen in animals treated with the HMD/INS-FUR vector after 2 months.

**Figure 17a** represents a liver of a control NOD mouse stained with immunoperoxidase for insulin. There is no evidence of insulin storage in the animals as would be expected. By comparison it can be seen in **Figure 17b****,** which is a section of a liver of an animal transduced with the HMD/INS-FUR vector that there are a large number of the cells in the liver have been converted into cells that stain positively for insulin. The insulin-containing cells are not isolated to the area surrounding the portal circulation, but are scattered throughout the liver tissue.

### Summary Discussion

The current study demonstrates a novel microsurgical technique used herein to transduce diabetic animals with a lentiviral vector carrying INS-FUR. Transduced animals processed, stored and secreted insulin in a glucose-regulated manner, resulting in the permanent amelioration of hyperglycaemia. Remission of diabetes due to the secretion of insulin from residual β-cells was discounted as insulin positive pancreatic β-cells were rarely observed and levels of rat c-peptide were negligible during IVGTT. Strikingly, transduction of INS-FUR alone induced expression of several β-cell transcription factors and instructed hepatocyte differentiation along a pancreatic lineage.

Secretion of insulin from liver cells without induction of storage is via the constitutive pathway. It has been shown that secretory granules developed in the human liver cell line Huh7, (that endogenously express NeuroD) on transfection of insulin alone, resulting in regulated expression of insulin and correction of diabetes. The expression of NeuroD by Huh7 cells and the concomitant storage of insulin is related to the fact that liver and pancreas derive from common progenitor cells and transdifferentiation between liver and pancreas has been illustrated. It was interesting in the current study that HMD alone could stimulate transcription of PDX-1 and Neuro-D, whereas insulin was required for protein synthesis and the expression of a repertoire of pancreatic markers. Hepatocytes have a unique predisposition to activate expression of pancreatic markers due to pre-existing hepatocyte nuclear factors 3β and 1α, which have been shown to control PDX-1 expression in β-cells. Given the pivotal role that PDX-1 and other transcription factors play in the generation of the β-cell phenotype, it is perhaps not surprising that the ability of our methodology to permanently reverse chemically-induced diabetes resides in the induction of β-cell transcription factors. However, whilst there was some pancreatic transdifferentiation, it was incomplete as transcription factors late in the hierarchy; such as Nkx 6.1 and Pax 6; were not expressed, nor was there expression of rodent insulin, PC2, pancreatic polypeptide, or exocrine markers. Thus, the problems encountered with non-selective pancreatic endocrine transdifferentiation produced on expression of PDX-1 such as the development of hepatitis due to secretion of exocrine enzymes was not witnessed. Also, as the transdifferentiation was only partial it is anticipated that this procedure will be capable of reversing diabetes on an autoimmune background.

The liver also has a high regenerative capacity and a large number of pluripotent progenitor cells, some of which can be activated by cellular insult to differentiate into cells expressing pancreatic markers. Thus, the microenvironment of STZ-induced hyperglycaemia may also have facilitated expression of the transcription factors in transduced animals. It is possible that the development of insulin-secreting cells in this study resulted from stem cells exposed to hyperglycaemic conditions.

The prevention of hypoglycaemia in STZ-rats after transduction with HMD/INS-FUR may be attributable, in part, to the presence of glucagon and somatostatin expressing cells providing counter-regulatory mechanisms. Unlike other studies that have engineered β-cell neogenesis from liver cells, the insulin-containing cells were not isolated to the area surrounding the portal circulation, but were scattered throughout the liver tissue. Insulin storage following acid/ ethanol extraction was 24,500 ng per liver, compared to 61,000 ng for a non-diabetic rat pancreas. This figure represents 40% of the insulin content of a rat pancreas and falls within the clinical limits (Ryan EA et al. Successful islet transplantation. Continued insulin reverse provides long-term glycemic control. Diabetes, 51: 2148-57 (2002) required for insulin independence. The material stored in the granules was predominately fully processed insulin..

The current study also demonstrates that the multiply-attenuated lentiviral vector pHR'CMVGFP can efficiently transduce non-dividing hepatocytes, both *in vitro* and in *vivo,* in the absence of additional growth factors or hepatic toxins to promote cell division. We obtained a significant improvement in transduction efficiency of up to 87% compared to 68% obtained by Zahler et al (Zahler, M. H. The application of a lentiviral vector for gene transfer in fetal hepatocytes. J. Gene Med. 2, 186-193 (2000) who used this vector to transduce non-dividing fetal human hepatocytes. The improvement in transduction efficiency may be attributable, in part, to the isolation of hepatocytes by perfusion of both the PV and AA as opposed to the PV alone, which is the most widely employed procedure (Berry, M. N. & Friend, D. S. High-yield preparation of isolated rat liver parenchymal cells. J. Cell Biol. 43, 506-520 (1969).

Most previous studies on the in vivo delivery of genes to hepatocytes using lentiviral vectors have indicated that cell cycling greatly improves transduction efficiency. Follenzi et al (Follenzi, A., Sabatino, G., Lombardo, A., Boccaccio, c., & Naldini, L. Efficient gene delivery and targeted expression to hepatocytes in vivo by improved lentiviral vectors. Hum. Gene. Ther. 13, 234-260 (2002) have delivered a lentiviral vector via the tail vein of rats, resulted in 20% transduction of non-dividing hepatocytes over a period of 13 weeks. However, due to the route of injection, the vector was expressed in several tissues. In the current study, we delivered INS-FUR in the HMD hybrid vector; which has been shown to efficiently transduce CD34⁺ haematopoetic cells with high levels of transgene expression; to the portal circulation of STZ-diabetic Wistar rats by FFO. PCR analysis also indicated that expression of INS-FUR was isolated to the liver tissue. Although the promoter used was not specific for hepatocyte-directed expression, the FFO procedure used may have efficiently confined the transgene in the liver tissue. Delivery of an insulin gene to the liver of diabetic rats using a recombinant adeno-associated vector via the portal circulation resulted in expression mainly in the liver, however reversal was dependent on vector dose and animals incurred significant liver damage (Park Y. I. et al. Safety and efficacy of adeno-associated viral vector-mediated insulin gene transfer via portal vein to the livers of streptozotocin-induced diabetic Sprague-Dawley rats. J. Gene Med. 7, 621-629 (2005).

Previous attempts to surgically transfuse vectors via the portal circulation have experienced major shortcomings. In studies utilizing an asanguineous perfusion method, which involves an incision in the inferior vena cava and subsequent insertion of a cannula, there is a potential risk of inferior vena cava narrowing and pelvic limb thrombosis. The use of portal branch ligation, to induce significant hepatocyte division, would likewise not be suitable for clinical applications. By comparison, delivery of the lentiviral vector via the FFO technique described in this study resulted in transduction of 75% of hepatocytes without inducing significant cell division or elevation of liver function enzymes.

The present invention also describes an improved method for hepatocyte isolation from rat liver and improved growth conditions for effective long-term transduction of hepatocytes that will be useful for *ex vivo* gene therapy. The present invention also describes a surgical technique for delivery of lentiviral vectors to rat livers that is much less invasive than a partial hepatectomy and leads to the efficient transduction of up to 75% of the liver cells, without the requirement for significant cell division. The present invention also relates to use of the surgical technique to deliver lentivector-encoded furin-cleavable insulin (INS-FUR, which enables liver to cleave proinsulin to mature insulin) to the livers of diabetic Wistar rats, which has resulted in normalization of blood glucose levels for 500 days, significant insulin storage in discrete granules, restoration of glucose tolerance, without inducing liver damage. The method of the invention has also been demonstrated herein to bring about stable reversal of diabetes by the transduction of only insulin and maintained expression of over 2 months in NOD mice.

In summary, the present inventors have developed a unique system, which has resulted in long-term reversal of diabetes by expression of INS-FUR alone. The method of gene delivery may also be applicable to other liver diseases associated with the absence of a functional gene, such as familial hypercholesterolemia, hemophilia, Crigler-Najjar syndrome.

## Claims

1. A lentiviral vector comprising a nucleic acid molecule encoding a polypeptide having the biological activity of insulin, factor VIII, factor IX, alpha 1 antitrypsin, hepatic bilirubin UDP-glucuronyltransferase or low density lipoprotein receptor, or a precursor thereof, for use in the treatment of a liver disorder or diabetes mellitus, by method comprising:
(i) inhibiting blood flow to the liver of said mammal;
(ii) transfusing liver cells of said mammal with a lentiviral vector, the vector comprising said nucleic acid molecule;
(iii) restoring blood flow to and from said liver.

2. A lentiviral vector according to claim 1, wherein the polypeptide has the biological activity of insulin and the lentivirus vector is for the treatment of type 1 diabetes mellitus.

3. A lentivirus vector according to claim 1 or 2, wherein the lentivirus vector is selected from the group consisting of HIV-MSCV hybrid viral vectors.

4. A lentivirus vector according to any one of the preceding claims, wherein the nucleic acid molecule comprises, or is in operable association with, a promoter and/or enhancer sequence.

5. A lentivirus vector according to any one of the preceding claims, wherein the nucleic acid encodes a furin-cleavable human insulin or human proinsulin sequence.

6. A lentivirus vector according to any one of the preceding claims, wherein inhibition of blood flow to the liver comprises ligating or clamping the right adrenal vein of said liver, successively clamping the infrahepatic vena cava, the hepatic artery, the portal vein, and the suprahepatic vena cava of said mammal.

## Patentansprüche

1. Ein Lentivirusvektor, umfassend ein Nukleinsäuremolekül, kodierend für ein Polypeptid mit der biologischen Aktivität von Insulin, Faktor VIII, Faktor IX, Alpha 1 Antitrypsin, hepatischer Bilirubin UDP-Glucuronyltransferase oder Low Density Lipoproteinrezeptor, oder eines Vorläufers davon, zur Verwendung bei der Behandlung einer Lebererkrankung oder Diabetes Mellitus, mit einer Methode, umfassend:
(i) Verhinderung des Blutflusses zu der Leber des besagten Säugers;
(ii) Transfundieren der Leberzellen des besagten Säugers mit einem Lentivirusvektor, wobei der Vektor die besagten Nukleinsäuremoleküle umfasst;
(iii) Herstellen des Blutflusses zu und von der besagten Leber.

2. Ein Lentivirusvektor nach Anspruch 1, wobei das Polypeptid die biologische Aktivität von Insulin hat und der Lentivirus für die Behandlung von Typ 1 Diabetes Mellitus vorgesehen ist.

3. Ein Lentivirusvektor nach Anspruch 1 oder 2, wobei der Lentivirusvektor ausgewählt ist aus der Gruppe bestehend aus HIV-MSCV viralen Hybridvektoren.

4. Ein Lentivirusvektor nach einem der vorhergehenden Ansprüche, wobei das Nukleinsäuremolekül umfasst oder in einer Funktionseinheit ist mit einem Promotor und/oder einer Enhancersequenz.

5. Ein Lentivirusvektor nach einem der vorhergehenden Ansprüche, wobei die Nukleinsäure für ein furin-schneidendes Humaninsulin oder humane Proinsulinsequenz kodiert.

6. Ein Lentivirusvektor nach einem der vorhergehenden Ansprüche, wobei die Verhinderung des Blutflusses zu der Leber das Abbinden oder Klammern der rechten Nebennierenvene besagter Leber umfasst, das erfolgreiche Klammern der infrahepatischen Vena Cava, der hepatischen Arterie, der Portalvene, und der suprahepatischen Vena Cava von besagtem Säuger.

## Revendications

1. Vecteur lentiviral comprenant une molécule d'acide nucléique codant un polypeptide ayant l'activité biologique de l'insuline, du facteur VIII, du facteur IX, de l'alpha-1-antitrypsine, de la bilirubine UDP-glucuronyltransférase hépatique ou du récepteur des lipoprotéines de basse densité, ou un précurseur de ceux-ci, pour utiliser dans le traitement d'une pathologie hépatique ou le diabète sucré selon un procédé comprenant :
(i) l'inhibition du flux sanguin vers le foie dudit mammifère ;
(ii) la transfusion de cellules du foie dudit mammifère avec un vecteur lentiviral, le vecteur comprenant ladite molécule d'acide nucléique ;
(iii) le rétablissement du flux sanguin vers et à partir du foie.

2. Vecteur lentiviral selon la revendication 1, dans lequel le polypeptide a l'activité biologique de l'insuline et le vecteur lentiviral est destiné au traitement du diabète sucré de type 1.

3. Vecteur lentiviral selon la revendication 1 ou 2, dans lequel le vecteur lentiviral est choisi dans le groupe constitué des vecteurs viraux hybrides VIH-MSCV.

4. Vecteur lentiviral selon l'une quelconque des revendications précédentes, dans lequel la molécule d'acide nucléique comprend, ou est associée de manière fonctionnelle à, une séquence promoteur et/ou amplificateur.

5. Vecteur lentiviral selon l'une quelconque des revendications précédentes, dans lequel l'acide nucléique code une séquence de proinsuline humaine ou d'insuline humaine clivable par la furine.

6. Vecteur lentiviral selon l'une quelconque des revendications précédentes, dans lequel l'inhibition du flux sanguin vers le foie comprend la ligature ou le clampage de la veine surrénale droite dudit foie, le clampage successif de la veine cave infrahépatique, de l'artère hépatique, de la veine porte et de la veine cave suprahépatique dudit mammifère.
